# EUROPEAN PATENT SPECIFICATION

(11) **EP 3 633 780 B1**
(45) Date of publication and mention of the grant of the patent: **07.10.2026**
(21) Application number: 17911418.6
(22) Date of filing: 23.06.2017
(51) Int. Cl.: H01M 10/0566, H01M 10/0569, H01M 10/0525, H01M 10/0567

(54) **NON-AQUEOUS ELECTROLYTE FOR LITHIUM ION BATTERY AND LITHIUM ION BATTERY**
WASSERFREIER ELEKTROLYT FÜR LITHIUM-IONEN-BATTERIE SOWIE LITHIUM-IONEN-BATTERIE
ÉLECTROLYTE NON AQUEUX POUR BATTERIE AU LITHIUM-ION ET BATTERIE AU LITHIUM-ION

(30) Priority: 27.05.2017 CN 201710393438
(43) Date of publication of application: 08.04.2020
(73) Proprietor: Shenzhen Capchem Technology Co., Ltd, Shenzhen, Guangdong 518118 (CN)
(72) Inventor: DENG, Zhaohui, Shenzhen Guangdong 518000 (CN); HU, Shiguang, Shenzhen Guangdong 518000 (CN); LIN, Muchong, Shenzhen Guangdong 518000 (CN); SHI, Qiao, Shenzhen Guangdong 518000 (CN)
(74) Representative: Herrero & Asociados, S.L.
(86) International application number: PCT/CN2017/089664
(87) International publication number: WO 2018/218714

(56) References cited:
- EP-A1- 3 483 972
- EP-A1- 3 618 164
- EP-A1- 3 618 165
- CN-A- 105 633 461
- GB-A- 1 147 540
- US-A1- 2013 089 778
- US-B1- 6 174 629

## Description

### Technical field

The invention relates to the field of lithium ion batteries, more particularly, to a non-aqueous electrolyte for lithium ion battery and lithium ion battery.

### Background

With the development of new energy vehicles and power energy storage, people have higher requirements for the performances of power lithium ion batteries, which requires further development of lithium ion batteries, so as to better meet the demand. Increasing the working voltage of battery is an effective method to improve the energy density of battery, but at the same time, the increasing of the working voltage of battery tends to degrade the performances of battery. This is because the existing common commercial electrolyte (such as carbonate electrolyte) will undergo oxidative decomposition when working at a voltage of 4.5-5V. On the one hand, some oxidation products of the electrolyte will deposit on the electrode surface, increasing the impedance of the battery and seriously deteriorating the electrochemical performance of the battery. On the other hand, the gas generated by the oxidative decomposition of electrolyte will cause the battery to swell, causing hidden trouble of safety.

In order to improve the stability of carbonate electrolyte under high voltage (4.5 ~ 5V), a practical method is to add fluorinated solvent to this kind of electrolyte to inhibit the electrolyte from decomposing too fast under high voltage condition. According to a literature report (Electrochemistry Communications 44 (2014) 34-37), fluorocarbonates can significantly improve the high-temperature cycle performance of high-voltage lithium ion batteries. However, repeated tests and studies by researchers in this field have found that batteries using fluorocarbonates alone generate serious gas when stored at high temperature, which has potential safety hazards. On the other hand, patent CN105633461 provides a secondary battery using dicarbonic ester, dicarboxylic or similar compound, capable of improving cycle characteristics, conservation characteristics, and load characteristics. And, patent WO2016/025589 discloses that adding fluorocarboxylic acid ester as electrolyte solvent to improve the high-temperature cycle performance of high-voltage lithium ion batteries. However, while fluorocarboxylic acid ester improves the cycle performance of the battery, there are problems in compatibility between fluorocarboxylic acid ester and carbon cathode material, which will cause the battery to inflate during high temperature cycle, bring potential safety hazards and deteriorate the battery performance.

### Summary

The application provides a non-aqueous electrolyte for lithium ion battery with good high-temperature cycle performance and less gas generation in high-temperature storage, aims to solve the problems that the existing non-aqueous electrolyte for lithium ion battery containing fluorinated solvent generates serious gas expansion when improving the high-temperature cycle performance and affects the high-temperature safety performance of the battery.

The application aims to provide a lithium ion battery containing the above-mentioned non-aqueous electrolyte for lithium ion battery.

The invention is realized as the following: a non-aqueous electrolyte for lithium ion battery, comprising a compound A and a compound B, wherein the compound A is at least one of compounds represented by the following structural formula I and formula II; the compound B is a compound represented by the following structural formula IV,

formula I: R₁-COO-R₂;

formula II: R₃-OCOO-R₄;

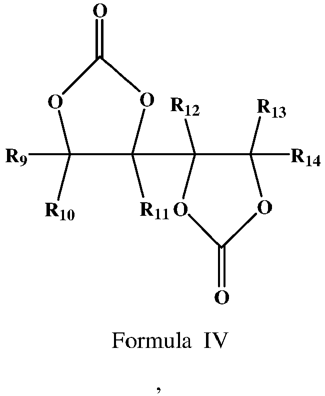

in formula I, R₁ is a hydrogen atom, a C1-C5 hydrocarbon group or a C1-C5 fluorohydrocarbon group, R₂ is a C1-C5 hydrocarbon group or a C1-C5 fluorohydrocarbon group, and at least one of R₁ and R₂ contains a fluorine atom;
in formula II, R₃ and R₄ are each independently selected from C1-C5 hydrocarbon group or C1-C5 fluorohydrocarbon group, and at least one of R₃ and R₄ contains a fluorine atom;
in formula IV, R₉, R₁₀, R₁₁, R₁₂, R₁₃ and R₁₄ are each independently selected from one of hydrogen atom, fluorine atom or C1-C5 group.

Preferably, in formula IV, the C1-C5 group is selected from a hydrocarbon group, fluorinated hydrocarbon group, oxygen-containing hydrocarbon group, silicon-containing hydrocarbon group, and cyano-substituted hydrocarbon group.

Preferably, R₉, R₁₀, R₁₁, R₁₂, R₁₃ and R₁₄ are each independently selected from a hydrogen atom, fluorine atom, methyl group, ethyl group, methoxyl group, ethyoxyl group, trimethylsiloxy group, cyano group or trifluoromethyl group.

Preferably, the compound B comprises one or more of compounds 1-9 represented by the following structural formulae,

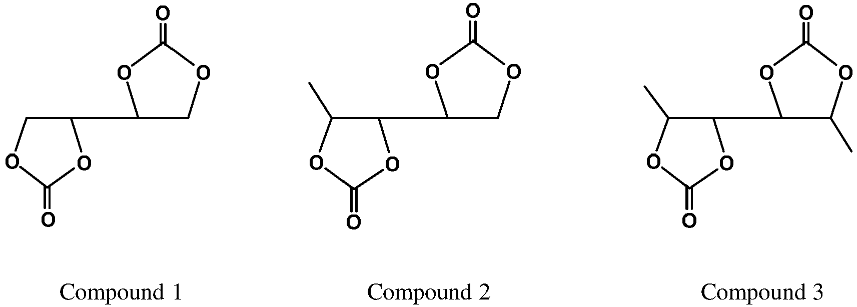

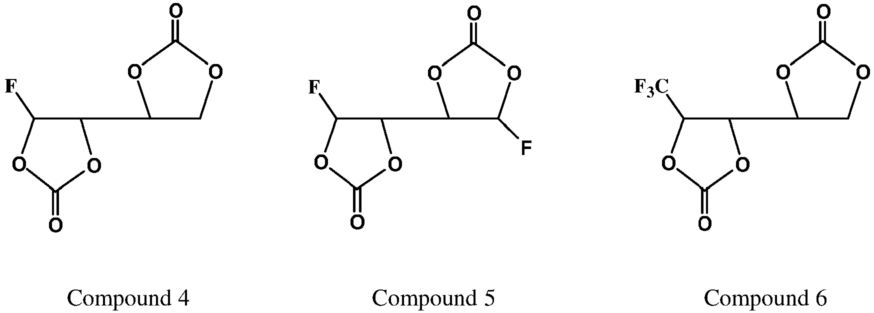

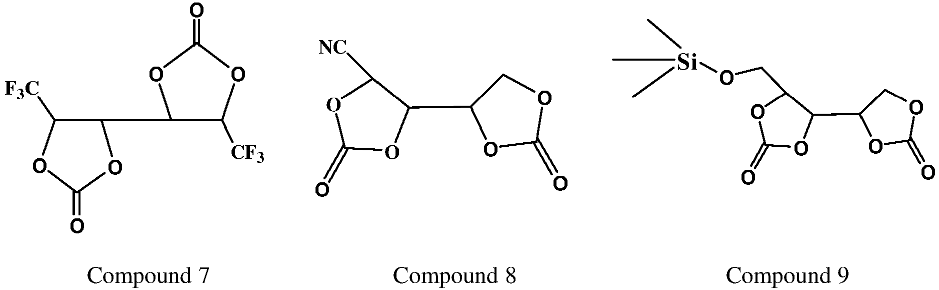

Preferably, the percentage mass content of the compound B is 0.1-5% based on the total mass of the non-aqueous electrolyte for lithium ion battery being 100%.

Preferably, the compound represented by formula I is selected from one or more of H₃CCOOCH₂CF₂H, H₃CH₂CCOOCH₂CF₂H, HF₂CH₂CCOOCH₃, HF₂CH₂CCOOCH₂CH₃, HF₂CH₂CH₂CCOOCH₂CH₃, H₃CCOOCH₂CH₂CF₂H, H₃CH₂CCOOCH₂CH₂CF₂H, CH₃COOCH₂CF₃, HCOOCH₂CHF₂, HCOOCH₂CF₃ and CH₃COOCH₂CF₂CF₂H.

Preferably, the compound represented by formula II is selected from one or more of CH₃OCOOCH₂CF₂H, CH₃OCOOCH₂CF₃, CH₃OCOOCH₂CF₂CF₂H, HCF₂CH₂OCOOCH₂CH₃ and CF₃CH₂OCOOCH₂CH₃.

Preferably, the compound A further comprises a compound represented by formula III, 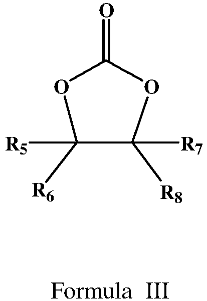 in formula III, R₅, R₆, R₇ and R₈ are each independently selected from hydrogen atom, fluorine atom, C1-C4 hydrocarbon group or C1-C4 fluorohydrocarbon group, and at least one of R₅, R₆, R₇ and R₈ contains a fluorine atom.

Preferably, the compound represented by formula III is selected from one or more of compounds 10-13 represented by the following structures,

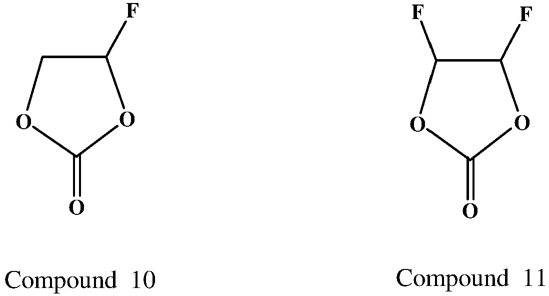

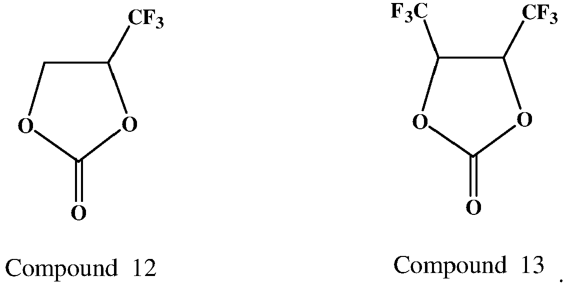

Preferably, the percentage mass content of the compound A is greater than 5% and less than 80% based on the total mass of the non-aqueous electrolyte for lithium ion battery being 100%.

Preferably, R₅ in formula III is fluorine, R₆, R₇ and R₈ are each independently selected from hydrogen atom, fluorine atom, C1-C4 hydrocarbon group or C1-C4 fluorohydrocarbon group, and R₆, R₇ and R₈ cannot be hydrogen atom at the same time; or R₅ is C1-C4 fluorine-containing hydrocarbon group, R₆, R₇ and R₈ are independently selected from hydrogen atom, fluorine atom, C1-C4 hydrocarbon group or C1-C4 fluorine-containing hydrocarbon group.

Preferably, the lithium ion non-aqueous electrolyte further comprises at least one of unsaturated cyclic carbonate compounds and sultone compounds.

More preferably, the unsaturated cyclic carbonate compound includes at least one of vinylene carbonate (VC) and vinyl ethylene carbonate (VEC).

More preferably, the sultone compound is selected from at least one of 1,3-propane sultone (PS), 1,4- butane sultone (BS), and 1,3-propene sultone (PST).

Preferably, the percentage mass content of the unsaturated cyclic carbonate compound is 0.1-5% based on the total mass of the non-aqueous electrolyte of the lithium ion battery being 100%; the percentage mass content of the sultone compound is 0.1-5% based on the total mass of the non-aqueous electrolyte of the lithium ion battery being 100%

Also provided is a non-aqueous electrolyte for lithium ion battery, comprising a compound A and a compound B, the compound A is a compound represented by the following structural formula III; the compound B is a compound represented by the following structural formula IV, 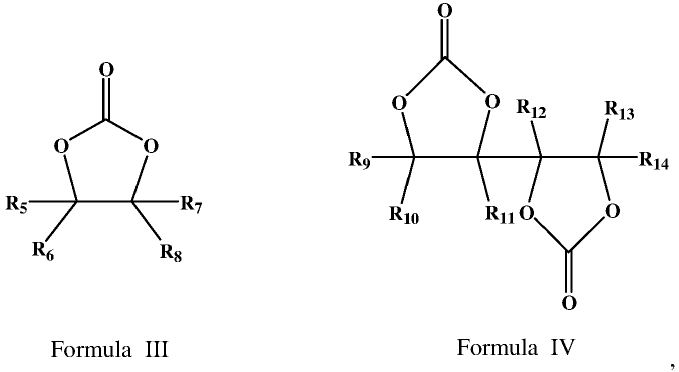
in formula III, R₅, R₆, R₇ and R₈ are each independently selected from hydrogen atom, fluorine atom, C1-C4 hydrocarbon group or C1-C4 fluorohydrocarbon group, and at least one of R₅, R₆, R₇ and R₈ contains a fluorine atom, and the compound represented by the structural formula III does not include fluoroethylene carbonate;
in formula IV, R₉, R₁₀, R₁₁, R₁₂, R₁₃ and R₁₄ are each independently selected from one of hydrogen atom, fluorine atom or C1-C5 group.

Preferably, the compound represented by formula III is selected from one or more of compounds 11-13 represented by the following structures, 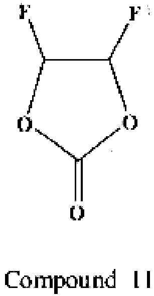 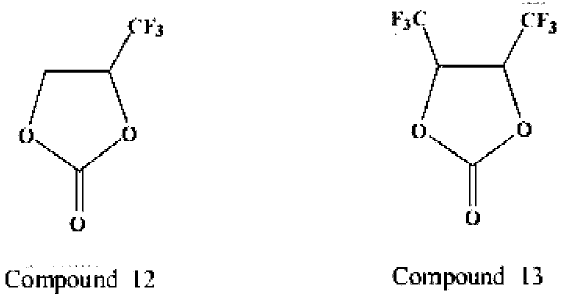 the compound B comprises one or more of compounds 1-9 represented by the following structural formulae, 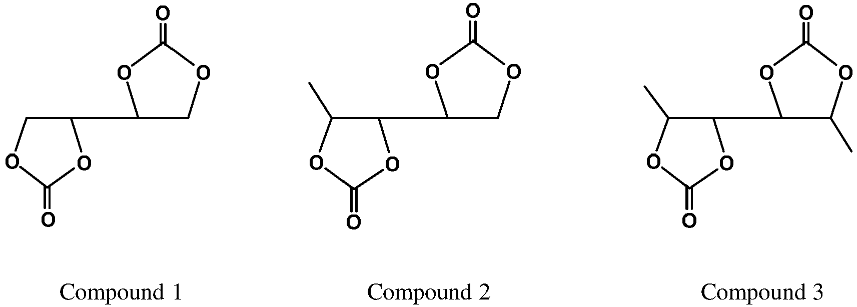 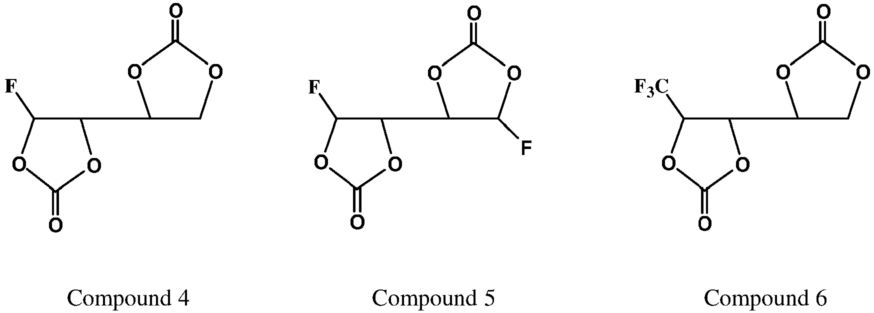 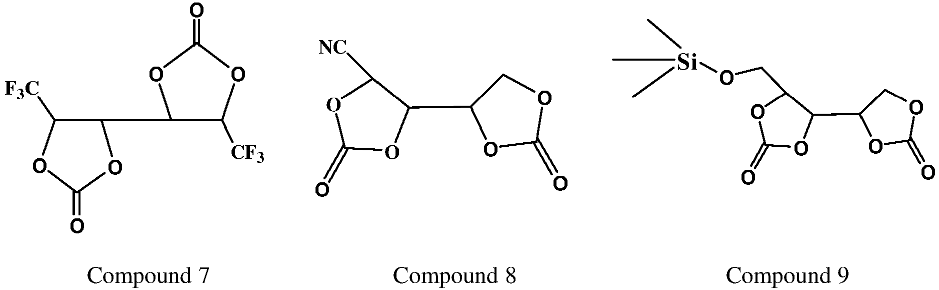 .

Preferably, the percentage mass content of the compound B is 0.1-5% based on the total mass of the non-aqueous electrolyte for lithium ion battery being 100%;
the percentage mass content of the compound A is greater than 5% and less than or equal to 80% based on the total mass of the non-aqueous electrolyte for lithium ion battery being 100%.

Preferably, the lithium ion non-aqueous electrolyte further comprises at least one of unsaturated cyclic carbonate compounds and sultone compounds;
the percentage mass content of the unsaturated cyclic carbonate compound is 0.1-5% based on the total mass of the non-aqueous electrolyte of the lithium ion battery being 100%; the percentage mass content of the sultone compound is 0.1-5% based on the total mass of the non-aqueous electrolyte of the lithium ion battery being 100%.

Also provided is a lithium ion battery, comprises a positive electrode, a negative electrode, a separator interposed between the positive electrode and the negative electrode, and an electrolyte, wherein the electrolyte is the non-aqueous electrolyte for lithium ion battery.

Preferably, the positive electrode comprises a positive electrode active material, and the positive electrode active material is at least one of LiNiₓCo_{y}Mn_{z}L_{(1-x-y-z)}O₂, LiCo_{x'}L_{(1-x')}O₂, LiNi_{x"}L'_{y'}Mn_{(2-x"-y')}O₄ and Li_{z'}MPO₄, wherein, L is at least one of Al, Sr, Mg, Ti, Ca, Zr, Zn, Si or Fe, 0≤x≤1, 0≤y≤1, 0≤z≤1, 0 < x+y+z≤1, 0<x'≤1, 0.3≤x"≤0.6, 0.01≤y'≤0.2, L' is at least one of Co, Al, Sr, Mg, Ti, Ca, Zr, Zn, Si and Fe; 0.5≤z'≤1, M is at least one of Fe, Mn and Co.

The non-aqueous electrolyte for lithium ion battery provided by the invention contains both the compound A and the compound B, the synergistic effect of compound A and compound B can ensure the thermal stability (high-temperature safety performance) of the negative electrode passivation film, and effectively improve the high-temperature cycle performance and the high-temperature storage performance of battery, and can also give consideration to the low-temperature performance of battery. The lithium ion battery containing the non-aqueous electrolyte has excellent high-temperature cycle performance, high-temperature storage performance and good low-temperature performance.

The lithium ion battery provided by the embodiments of the invention contains the non-aqueous electrolyte, so that the lithium ion battery has better high-temperature cycle performance, high-temperature storage performance and low-temperature performance.

### Detailed description of disclosed embodiment

In order to make the technical problems to be solved, technical solutions and beneficial effects more apparent and clearer, the present application will be described in further detail below with reference to embodiments. It should be understood that the specific embodiments described herein are only for the purpose of explaining the present invention and are not intended to limit the present invention.

In the embodiments of the invention, the names and their English abbreviations are as follows:
FEC: fluoroethylene carbonate;
DFEC: 1,2-difluoroethylene carbonate;
PC: propylene carbonate;
EC: ethylene carbonate;
DEC: diethyl carbonate.

The embodiment of the invention provides a non-aqueous electrolyte for lithium ion battery, comprising a compound A and a compound B, wherein the compound A is at least one of compounds represented by the following structural formula I, formula II and formula III; the compound B is a compound represented by the following structural formula IV,

formula I: R₁-COO-R₂;

formula II: R₃-OCOO-R₄;

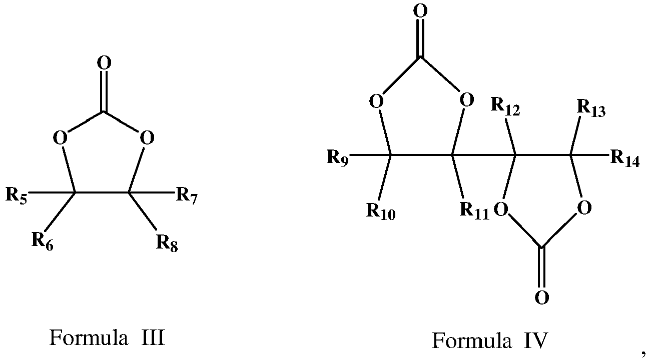

in formula I, R₁ is a hydrogen atom, a C1-C5 hydrocarbon group or a C1-C5 fluorohydrocarbon group, R₂ is a C1-C5 hydrocarbon group or a C1-C5 fluorohydrocarbon group, and at least one of R₁ and R₂ contains a fluorine atom;
in formula II, R₃ and R₄ are each independently selected from C1-C5 hydrocarbon group or C1-C5 fluorohydrocarbon group, and at least one of R₃ and R₄ contains a fluorine atom;
in formula III, R₅, R₆, R₇ and R₈ are each independently selected from hydrogen atom, fluorine atom, C1-C4 hydrocarbon group or C1-C4 fluorohydrocarbon group, and at least one of R₅, R₆, R₇ and R₈ contains a fluorine atom;
in formula IV, R₉, R₁₀, R₁₁, R₁₂, R₁₃ and R₁₄ are each independently selected from one of hydrogen atom, fluorine atom or C1-C5 group.

In the embodiment of the invention, C1-C5 refers to the number of carbon atoms being 1-5, similarly, C1-C4 refers to the number of carbon atoms being 1-4.

Preferably, the compound represented by formula I is selected from one or more of H₃CCOOCH₂CF₂H, H₃CH₂CCOOCH₂CF₂H, HF₂CH₂CCOOCH₃, HF₂CH₂CCOOCH₂CH₃, HF₂CH₂CH₂CCOOCH₂CH₃, H₃CCOOCH₂CH₂CF₂H, H₃CH₂CCOOCH₂CH₂CF₂H, CH₃COOCH₂CF₃, HCOOCH₂CHF₂, HCOOCH₂CF₃ and CH₃COOCH₂CF₂CF₂H, but is not limited thereto.

Preferably, the compound represented by formula II is selected from one or more of CH₃OCOOCH₂CF₂H, CH₃OCOOCH₂CF₃ (methyl 2,2,2-trifluoroethyl carbonate), CH₃OCOOCH₂CF₂CF₂H, HCF₂CH₂OCOOCH₂CH₃ and CF₃CH₂OCOOCH₂CH₃, but is not limited thereto.
in formula III, R₅, R₆, R₇ and R₈ are each independently selected from hydrogen atom, fluorine atom, C1-C4 hydrocarbon group or C1-C4 fluorohydrocarbon group, and at least one of R₅, R₆, R₇ and R₈ contains a fluorine atom;

Specifically, the compound represented by formula III can be fluoroethylene carbonate, or R₅ in formula III is fluorine, R₆, R₇ and R₈ are each independently selected from hydrogen atom, fluorine atom, C1-C4 hydrocarbon group or C1-C4 fluorohydrocarbon group, and R₆, R₇ and R₈ cannot be hydrogen atom at the same time; or R₅ is C1-C4 fluorine-containing hydrocarbon group, R₆, R₇ and R₈ are independently selected from hydrogen atom, fluorine atom, C1-C4 hydrocarbon group or C1-C4 fluorine-containing hydrocarbon group.

Preferably, the compound represented by formula III is selected from one or more of compounds 10-13 represented by the following structures, 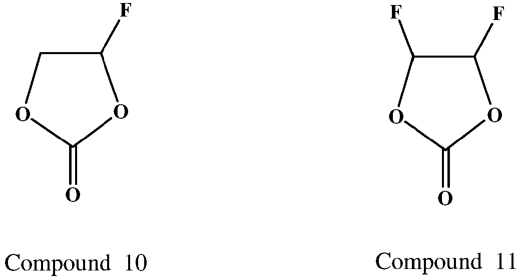 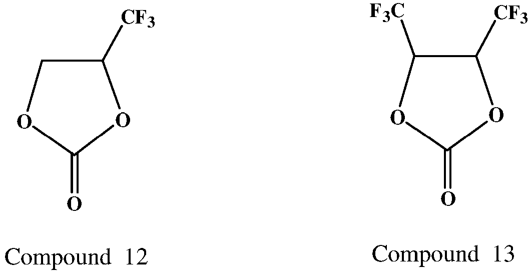 and is not limited thereto.

The above preferred structures of formula I, formula II and formula III have better effect of improving the oxidation decomposition potential of the electrolyte, and cooperate with the compound B more effectively to improve the high-temperature cycle performance and high-temperature storage performance of the electrolyte, meanwhile, can also give consideration to the low-temperature performance of the battery.

The content of the above compound A can be varied within a wide range. Preferably, based on the total mass of the non-aqueous electrolyte for lithium ion battery being 100%, the percentage mass content of the compound A is less than 80%, for example, it may be 1%, 2%, 5%, 6%, 8%, 10%, 15%, 20%, 30%, 40%, 50%, 60%, 70%, 80%. More preferably, the percentage mass content of the compound A is greater than 5% and less than or equal to 80%, more preferably, the percentage mass content of the compound A is 10-80%.

When compound A is the compound represented by formula III, and is not FEC (i.e., R₅ in the formula III is fluorine, R₆, R₇ and R₈ are each independently selected from hydrogen atom, fluorine atom, C1-C4 hydrocarbon group or C1-C4 fluorohydrocarbon group, and R₆, R₇ and R₈ cannot be hydrogen atom at the same time; Or R₅ is a C1-C4 fluoroalkyl group, R₆, R₇ and R₈ are independently selected from hydrogen atom, fluorine atom, C1-C4 hydrocarbyl group, or C1-C4 fluoroalkyl group), the content thereof can be varied within a wide range without any particular limitation. Preferably, based on the total mass of the non-aqueous electrolyte for lithium ion battery being 100%, the percentage mass content of the compound A is less than 80%; for example, it may be 1%, 2%, 5%, 6%, 8%, 10%, 15%, 20%, 30%, 40%, 50%, 60%, 70%, 80%. More preferably, the percentage mass content of the compound A is greater than 5% and less than or equal to 80%, more preferably, the percentage mass content of the compound A is 10-80%.

When the compound A is FEC, the percentage mass content of the compound A is greater than 5% and less than or equal to 80% based on the total mass of the non-aqueous electrolyte for lithium ion battery being 100%.

It is understood that if the non-aqueous electrolyte for lithium ion battery contains one of the above substances, the content is the content of the one substance; If the non-aqueous electrolyte for lithium ion battery contains a plurality of the above substances, the content is the sum of the contents of the plurality of substances.

The non-aqueous electrolyte for lithium ion battery provided by the embodiments of the invention contains a compound A (fluorinated solvent), and the compound A has better oxidation resistance than carbonate and can improve the oxidation decomposition potential of the electrolyte. In addition, the compound A (fluorocarbonate and fluorocarboxylate) can form a passivation film on the surface of the negative electrode and inhibit the decomposition reaction of the electrolyte. However, under the condition of high voltage (4.5-5V), the compound A produces serious gas when the battery is formed, stored at high temperature and cycled at high temperature, which deteriorates the battery performances and brings potential safety hazards. Specifically, in the high-temperature storage process of the battery, the thermal stability of the passivation film of the fluorocarbonate solvent represented by formula II and formula III is not ideal, a large amount of gas is generated, and the high-temperature storage performance of the battery is reduced. During the initial charge process of the battery, the fluorocarboxylic acid ester solvent represented by the formula I decomposes on the surface of the negative electrode and generates a large amount of gas, which leads to poor contact between electrode plates, thereby reducing the performances of the lithium ion battery.

In the embodiments of the invention, the compound B represented by the structural formula IV is added to the non-aqueous electrolyte of the lithium ion battery on the basis of the compound A, the two compounds works together to improve the high-temperature storage performance, high-temperature cycle performance and low-temperature performance of the lithium ion battery. Specifically, the compound B forms a dense passivation film on the negative electrode in the battery formation process, preventing the decomposition of fluorinated solvent molecules (the compound A), inhibiting the generation of gas by the fluorinated solvent, which can improve the thermal stability of the negative electrode passivation film, effectively enhance the high-temperature cycle performance and high-temperature storage performance of the battery, hence ensuring the high-temperature performance of the lithium ion battery. At the same time, the passivation film formed by decomposition of the compound B has lower impedance and less influence on impedance growth, so that the low-temperature performance of the lithium ion battery can also be considered. In the embodiments of the invention, the compound A and the compound B are used together, because when the compound A is subjected to the film forming reaction on the surface of the negative electrode, the compound B also participates in the film forming reactions of positive and negative electrodes, so that the passivation layer components of positive and negative electrodes comprise both the decomposition products of the compound A and the compound B, thereby effectively improving the interface conditions of the positive and negative electrodes. The synergistic effect of the two compounds use together is better than that of the simple addition of the two compounds used separately.

In the compound B, the C1-C5 group is selected from a hydrocarbon group, fluorinated hydrocarbon group, oxygen-containing hydrocarbon group, silicon-containing hydrocarbon group, and cyano-substituted hydrocarbon group.In the compound B, R₉, R₁₀, R₁₁, R₁₂, R₁₃ and R₁₄ are each independently selected from a hydrogen atom, fluorine atom, methyl group, ethyl group, methoxyl group, ethyoxyl group, trimethylsiloxy group, cyano group or trifluoromethyl group.

Preferably, the compound B comprises one or more of compounds 1-9 represented by the following structural formulae,

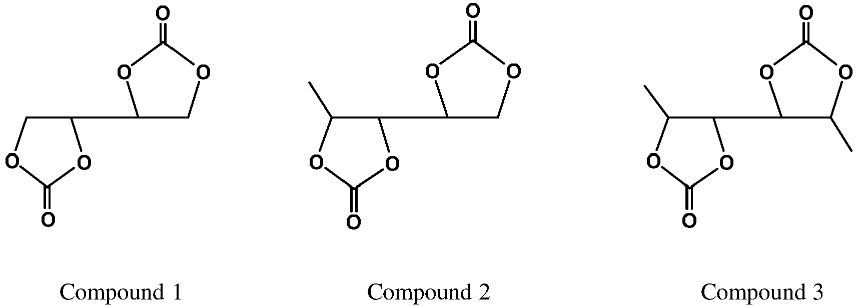

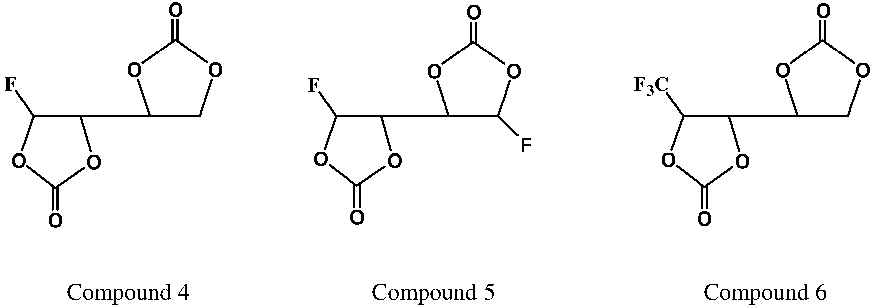

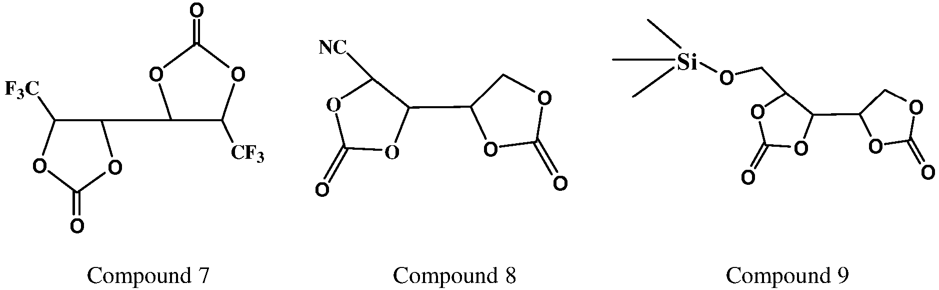

The preferred compound B can better cooperate with the compound A to endow the lithium ion battery with better comprehensive performances (high-temperature cycle performance, high-temperature storage performance and low-temperature performance).

The synthesis method of the compound B presented by formula IV is conventional, for example, the compound B can be prepared by ester exchange reaction between polyol (such as erythritol, xylitol, etc.) and carbonate (such as dimethyl carbonate, diethyl carbonate, vinyl carbonate, etc.) in the presence of basic catalyst. An example of the synthetic route is as follows:

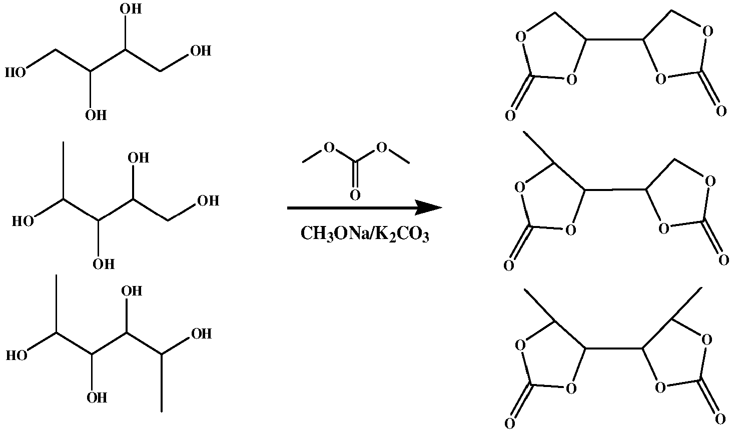

The fluorine-containing compound in compound B is prepared by: fluorinating the corresponding carbonate and mixture F₂/N₂, and then recrystallizing or purifying by column chromatography. An example of the synthetic route is as follows:

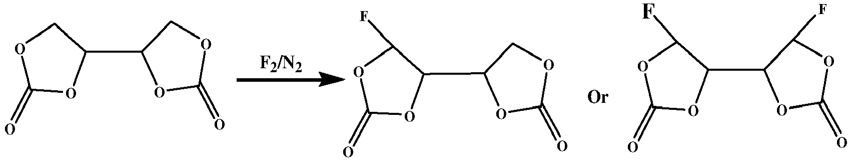

The cyano group-containing compound in compound B is prepared by: the chlorination reaction of the corresponding carbonate and sulfonyl chloride, then reacting with NaCN or KCN, and then recrystallizing or purifying by column chromatography. An example of the synthetic route is as follows:

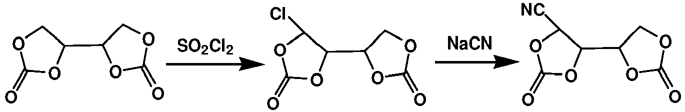

The trimethylsilanolate-containing compound in the compound B is prepared by: the substitution reaction of the corresponding hydroxy carbonate and silazane, then ecrystallizing or purifying by column chromatography. An example of the synthetic route is as follows:

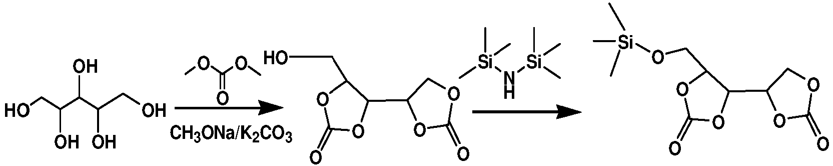

Preferably, the percentage mass content of the compound B is 0.1-5% based on the total mass of the non-aqueous electrolyte for lithium ion battery being 100%. For example, the percentage mass content of the compound B may be 0.1%, 0.2%, 0.4%, 0.5%, 0.6%, 0.8%, 1%, 1.2%, 1.5%, 1.8%, 2%, 2.5%, 3%, 3.5%, 4%, 4.5%, 5%. When the percentage mass content of the compound B is less than 0.1%, it is not conductive for the film formation at the negative electrode, and the effect of improving cycle performance is reduced; when the percentage mass content of the compound B is more than 5%, it is not conducive to uniform dissolution in the electrolyte, and the film formation at the electrode interface is quite thick, which will increase the battery impedance and deteriorate the battery performance.

Based on the above embodiments, it is preferred that the lithium ion non-aqueous electrolyte further comprises at least one of unsaturated cyclic carbonate compounds and sultone compounds.

Preferably, the unsaturated cyclic carbonate compound includes at least one of vinylene carbonate (VC) and vinyl ethylene carbonate (VEC). The sultone compound is selected from at least one of 1,3- propane sultone (PS), 1,4- butane sultone (BS), and 1,3-propene sultone (PST).

The content of unsaturated cyclic carbonate compound is 0.1-5% based on the total mass of the non-aqueous electrolyte for lithium ion battery being 100%.

The percentage mass content of sultone compound is 0.1-5% based on the total mass of the non-aqueous electrolyte for lithium ion battery being 100%.

The lithium ion non-aqueous electrolyte comprises lithium salt, and the lithium salt is not particularly limited in the present invention, various existing lithium salts can be used, for example, the lithium salt can be selected from one or more of LiPF₆, LiBOB and LiBF₄. The content of the lithium salt can vary within a wide range, and preferably, the content of the lithium salt in the non-aqueous electrolyte of the lithium ion battery is 0.1-15%.

Preferably, the non-aqueous electrolyte for lithium ion battery further comprises at least one of vinyl carbonate, propylene carbonate, butylene carbonate, dimethyl carbonate, diethyl carbonate, methyl ethyl carbonate and methyl propyl carbonate.

The non-aqueous electrolyte for lithium ion battery provided by the embodiments of the invention contains both the compound A and the compound B, the synergistic effect of compound A and compound B can ensure the thermal stability (high-temperature safety performance) of the negative electrode passivation film, and effectively improve the high-temperature cycle performance and the high-temperature storage performance of battery, and can also give consideration to the low-temperature performance of battery. The lithium ion battery containing the non-aqueous electrolyte has excellent high-temperature cycle performance, high-temperature storage performance and good low-temperature performance.

And, the embodiment of the invention also provides a lithium ion battery, comprising a positive electrode, a negative electrode, a separator interposed between the positive electrode and the negative electrode, and an electrolyte, wherein the electrolyte is the non-aqueous electrolyte for lithium ion battery.

Preferably, the positive electrode comprises a positive electrode active material, and the positive electrode active material is at least one of LiNiₓCo_{y}Mn_{z}L_{(1-x-y-z)}O₂, LiCo_{x'}L_{(1-x')}O₂, LiNi_{x"}L'_{y'}Mn_{(2-x"-y')}O₄, Li_{z} and MPO₄, wherein, L is at least one of Al, Sr, Mg, Ti, Ca, Zr, Zn, Si or Fe, 0≤x≤1, 0≤y≤1, 0≤z≤1, 0<x+y+z≤1, 0<x'≤1, 0.3≤x"≤0.6, 0.01≤y'≤0.2, L' is at least one of Co, Al, Sr, Mg, Ti, Ca, Zr, Zn, Si and Fe; 0.5≤z'≤1, M is at least one of Fe, Mn and Co.

In the embodiment of the present invention, the positive electrode, negative electrode and the separator are not specifically limited, they can be the conventional ones in the art.

The lithium ion battery provided by the embodiments of the invention contains the non-aqueous electrolyte, so that the lithium ion battery has better high-temperature cycle performance, high-temperature storage performance and low-temperature performance.

The following description will be made with reference to specific embodiments.

### Embodiment 1

A 4.4V LiNi_{0.5}Co_{0.2}Mn_{0.3}O₂/ artificial graphite battery, comprises a positive electrode, a negative electrode, a separator interposed between the positive electrode and the negative electrode, and an electrolyte, wherein the electrolyte is a non-aqueous electrolyte and comprises the following components in percentage by mass as shown in Table 1 of Embodiment 1, based on the total weight of the non-aqueous electrolyte taken as 100% by weight, and conventional lithium salts without showing.

### Embodiment 2

A 4.4V LiNi_{0.5}Co_{0.2}Mn_{0.3}O₂/ artificial graphite battery, comprises a positive electrode, a negative electrode, a separator interposed between the positive electrode and the negative electrode, and an electrolyte, wherein the electrolyte is a non-aqueous electrolyte and comprises the following components in percentage by mass as shown in Table 1 of Embodiment 2, based on the total weight of the non-aqueous electrolyte taken as 100% by weight, and conventional lithium salts without showing.

### Embodiment 3

A 4.4V LiNi_{0.5}Co_{0.2}Mn_{0.3}O₂/ artificial graphite battery, comprises a positive electrode, a negative electrode, a separator interposed between the positive electrode and the negative electrode, and an electrolyte, wherein the electrolyte is a non-aqueous electrolyte and comprises the following components in percentage by mass as shown in Table 1 of Embodiment 3, based on the total weight of the non-aqueous electrolyte taken as 100% by weight, and conventional lithium salts without showing.

### Embodiment 4

A 4.4V LiNi_{0.5}Co_{0.2}Mn_{0.3}O₂/ artificial graphite battery, comprises a positive electrode, a negative electrode, a separator interposed between the positive electrode and the negative electrode, and an electrolyte, wherein the electrolyte is a non-aqueous electrolyte and comprises the following components in percentage by mass as shown in Table 1 of Embodiment 4, based on the total weight of the non-aqueous electrolyte taken as 100% by weight, and conventional lithium salts without showing.

### Embodiment 5

A 4.4V LiNi_{0.5}Co_{0.2}Mn_{0.3}O₂/ artificial graphite battery, comprises a positive electrode, a negative electrode, a separator interposed between the positive electrode and the negative electrode, and an electrolyte, wherein the electrolyte is a non-aqueous electrolyte and comprises the following components in percentage by mass as shown in Table 1 of Embodiment 5, based on the total weight of the non-aqueous electrolyte taken as 100% by weight, and conventional lithium salts without showing.

### Embodiment 6

A 4.4V LiNi_{0.5}Co_{0.2}Mn_{0.3}O₂/ artificial graphite battery, comprises a positive electrode, a negative electrode, a separator interposed between the positive electrode and the negative electrode, and an electrolyte, wherein the electrolyte is a non-aqueous electrolyte and comprises the following components in percentage by mass as shown in Table 1 of Embodiment 6, based on the total weight of the non-aqueous electrolyte taken as 100% by weight, and conventional lithium salts without showing.

### Embodiment 7

A 4.4V LiNi_{0.5}Co_{0.2}Mn_{0.3}O₂/ artificial graphite battery, comprises a positive electrode, a negative electrode, a separator interposed between the positive electrode and the negative electrode, and an electrolyte, wherein the electrolyte is a non-aqueous electrolyte and comprises the following components in percentage by mass as shown in Table 1 of Embodiment 7, based on the total weight of the non-aqueous electrolyte taken as 100% by weight, and conventional lithium salts without showing.

### Embodiment 8

A 4.4V LiNi_{0.5}Co_{0.2}Mn_{0.3}O₂/ artificial graphite battery, comprises a positive electrode, a negative electrode, a separator interposed between the positive electrode and the negative electrode, and an electrolyte, wherein the electrolyte is a non-aqueous electrolyte and comprises the following components in percentage by mass as shown in Table 1 of Embodiment 8, based on the total weight of the non-aqueous electrolyte taken as 100% by weight, and conventional lithium salts without showing.

### Embodiment 9

A 4.4V LiNi_{0.5}Co_{0.2}Mn_{0.3}O₂/ artificial graphite battery, comprises a positive electrode, a negative electrode, a separator interposed between the positive electrode and the negative electrode, and an electrolyte, wherein the electrolyte is a non-aqueous electrolyte and comprises the following components in percentage by mass as shown in Table 1 of Embodiment 9, based on the total weight of the non-aqueous electrolyte taken as 100% by weight, and conventional lithium salts without showing.

### Embodiment 10

A 4.4V LiNi_{0.5}Co_{0.2}Mn_{0.3}O₂/ artificial graphite battery, comprises a positive electrode, a negative electrode, a separator interposed between the positive electrode and the negative electrode, and an electrolyte, wherein the electrolyte is a non-aqueous electrolyte and comprises the following components in percentage by mass as shown in Table 1 of Embodiment 10, based on the total weight of the non-aqueous electrolyte taken as 100% by weight, and conventional lithium salts without showing.

### Embodiment 11

A 4.4V LiNi_{0.5}Co_{0.2}Mn_{0.3}O₂/ artificial graphite battery, comprises a positive electrode, a negative electrode, a separator interposed between the positive electrode and the negative electrode, and an electrolyte, wherein the electrolyte is a non-aqueous electrolyte and comprises the following components in percentage by mass as shown in Table 1 of Embodiment 11, based on the total weight of the non-aqueous electrolyte taken as 100% by weight, and conventional lithium salts without showing.

### Embodiment 12

A 4.4V LiNi_{0.5}Co_{0.2}Mn_{0.3}O₂/ artificial graphite battery, comprises a positive electrode, a negative electrode, a separator interposed between the positive electrode and the negative electrode, and an electrolyte, wherein the electrolyte is a non-aqueous electrolyte and comprises the following components in percentage by mass as shown in Table 1 of Embodiment 12, based on the total weight of the non-aqueous electrolyte taken as 100% by weight, and conventional lithium salts without showing.

### Embodiment 13

A 4.4V LiNi_{0.5}Co_{0.2}Mn_{0.3}O₂/ artificial graphite battery, comprises a positive electrode, a negative electrode, a separator interposed between the positive electrode and the negative electrode, and an electrolyte, wherein the electrolyte is a non-aqueous electrolyte and comprises the following components in percentage by mass as shown in Table 1 of Embodiment 13, based on the total weight of the non-aqueous electrolyte taken as 100% by weight, and conventional lithium salts without showing.

### Embodiment 14

A 4.2V LiNi_{0.8}Co_{0.15}Al_{0.05}O₂/ Si-C battery, comprises a positive electrode, a negative electrode, a separator interposed between the positive electrode and the negative electrode, and an electrolyte, wherein the electrolyte is a non-aqueous electrolyte and comprises the following components in percentage by mass as shown in Table 3 of Embodiment 14, based on the total weight of the non-aqueous electrolyte taken as 100% by weight, and conventional lithium salts without showing.
s

### Embodiment 15

A 4.2V LiNi_{0.8}Co_{0.15}Al_{0.05}O₂/ Si-C battery, comprises a positive electrode, a negative electrode, a separator interposed between the positive electrode and the negative electrode, and an electrolyte, wherein the electrolyte is a non-aqueous electrolyte and comprises the following components in percentage by mass as shown in Table 3 of Embodiment 15, based on the total weight of the non-aqueous electrolyte taken as 100% by weight, and conventional lithium salts without showing.

### Embodiment 16

A 4.2V LiNi_{0.8}Co_{0.15}Al_{0.05}O₂/ Si-C battery, comprises a positive electrode, a negative electrode, a separator interposed between the positive electrode and the negative electrode, and an electrolyte, wherein the electrolyte is a non-aqueous electrolyte and comprises the following components in percentage by mass as shown in Table 3 of Embodiment 16, based on the total weight of the non-aqueous electrolyte taken as 100% by weight, and conventional lithium salts without showing.

### Embodiment 17

A 4.2V LiNi_{0.8}Co_{0.15}Al_{0.05}O₂/ Si-C battery, comprises a positive electrode, a negative electrode, a separator interposed between the positive electrode and the negative electrode, and an electrolyte, wherein the electrolyte is a non-aqueous electrolyte and comprises the following components in percentage by mass as shown in Table 3 of Embodiment 17, based on the total weight of the non-aqueous electrolyte taken as 100% by weight, and conventional lithium salts without showing.

### Comparative Example 1

A 4.4V LiNi_{0.5}Co_{0.2}Mn_{0.3}O₂/ artificial graphite battery, comprises a positive electrode, a negative electrode, a separator interposed between the positive electrode and the negative electrode, and an electrolyte, wherein the electrolyte is a non-aqueous electrolyte and comprises the following components in percentage by mass as shown in Table 1 of Comparative Example 1, based on the total weight of the non-aqueous electrolyte taken as 100% by weight, and conventional lithium salts without showing.

### Comparative Example 2

A 4.4V LiNi_{0.5}Co_{0.2}Mn_{0.3}O₂/ artificial graphite battery, comprises a positive electrode, a negative electrode, a separator interposed between the positive electrode and the negative electrode, and an electrolyte, wherein the electrolyte is a non-aqueous electrolyte and comprises the following components in percentage by mass as shown in Table 1 of Comparative Example 2, based on the total weight of the non-aqueous electrolyte taken as 100% by weight, and conventional lithium salts without showing.

### Comparative Example 3

A 4.4V LiNi_{0.5}Co_{0.2}Mn_{0.3}O₂/ artificial graphite battery, comprises a positive electrode, a negative electrode, a separator interposed between the positive electrode and the negative electrode, and an electrolyte, wherein the electrolyte is a non-aqueous electrolyte and comprises the following components in percentage by mass as shown in Table 1 of Comparative Example 3, based on the total weight of the non-aqueous electrolyte taken as 100% by weight, and conventional lithium salts without showing.

### Comparative Example 4

A 4.4V LiNi_{0.5}Co_{0.2}Mn_{0.3}O₂/ artificial graphite battery, comprises a positive electrode, a negative electrode, a separator interposed between the positive electrode and the negative electrode, and an electrolyte, wherein the electrolyte is a non-aqueous electrolyte and comprises the following components in percentage by mass as shown in Table 1 of Comparative Example 4, based on the total weight of the non-aqueous electrolyte taken as 100% by weight, and conventional lithium salts without showing.

### Comparative Example 5

A 4.4V LiNi_{0.5}Co_{0.2}Mn_{0.3}O₂/ artificial graphite battery, comprises a positive electrode, a negative electrode, a separator interposed between the positive electrode and the negative electrode, and an electrolyte, wherein the electrolyte is a non-aqueous electrolyte and comprises the following components in percentage by mass as shown in Table 1 of Comparative Example 5, based on the total weight of the non-aqueous electrolyte taken as 100% by weight, and conventional lithium salts without showing.

### Comparative Example 6

A 4.4V LiNi_{0.5}Co_{0.2}Mn_{0.3}O₂/ artificial graphite battery, comprises a positive electrode, a negative electrode, a separator interposed between the positive electrode and the negative electrode, and an electrolyte, wherein the electrolyte is a non-aqueous electrolyte and comprises the following components in percentage by mass as shown in Table 1 of Comparative Example 6, based on the total weight of the non-aqueous electrolyte taken as 100% by weight, and conventional lithium salts without showing.

### Comparative Example 7

A 4.4V LiNi_{0.5}Co_{0.2}Mn_{0.3}O₂/ artificial graphite battery, comprises a positive electrode, a negative electrode, a separator interposed between the positive electrode and the negative electrode, and an electrolyte, wherein the electrolyte is a non-aqueous electrolyte and comprises the following components in percentage by mass as shown in Table 1 of Comparative Example 7, based on the total weight of the non-aqueous electrolyte taken as 100% by weight, and conventional lithium salts without showing.

### Comparative Example 8

A 4.2V LiNi_{0.8}Co_{0.15}Al_{0.05}O₂/ Si-C battery, comprises a positive electrode, a negative electrode, a separator interposed between the positive electrode and the negative electrode, and an electrolyte, wherein the electrolyte is a non-aqueous electrolyte and comprises the following components in percentage by mass as shown in Table 3 of Comparative Example 8, based on the total weight of the non-aqueous electrolyte taken as 100% by weight, and conventional lithium salts without showing.

### Comparative Example 9

A 4.2V LiNi_{0.8}Co_{0.15}Al_{0.05}O₂/ Si-C battery, comprises a positive electrode, a negative electrode, a separator interposed between the positive electrode and the negative electrode, and an electrolyte, wherein the electrolyte is a non-aqueous electrolyte and comprises the following components in percentage by mass as shown in Table 3 of Comparative Example 9, based on the total weight of the non-aqueous electrolyte taken as 100% by weight, and conventional lithium salts without showing.

### Comparative Example 10

A 4.2V LiNi_{0.8}Co_{0.15}Al_{0.05}O₂/ Si-C battery, comprises a positive electrode, a negative electrode, a separator interposed between the positive electrode and the negative electrode, and an electrolyte, wherein the electrolyte is a non-aqueous electrolyte and comprises the following components in percentage by mass as shown in Table 3 of Comparative Example 10, based on the total weight of the non-aqueous electrolyte taken as 100% by weight, and conventional lithium salts without showing.

### Comparative Example 11

A 4.2V LiNi_{0.8}Co_{0.15}Al_{0.05}O₂/ Si-C battery, comprises a positive electrode, a negative electrode, a separator interposed between the positive electrode and the negative electrode, and an electrolyte, wherein the electrolyte is a non-aqueous electrolyte and comprises the following components in percentage by mass as shown in Table 3 of Comparative Example 11, based on the total weight of the non-aqueous electrolyte taken as 100% by weight, and conventional lithium salts without showing.

The Embodiments 1-13, Comparative Examples 1-7 (4.4V LiNi_{0.5}Co_{0.2}Mn_{0.3}O₂/ artificial graphite battery), Embodiments 14-17, Comparative Examples 8-11 (4.2V LiNi_{0.8}Co_{0.15}Al_{0.05}O₂/ Si-C battery) of the present invention were tested for performance, and the test parameters and test methods are as follows:
(1) High-temperature cycle performance is demonstrated by testing the capacity retention rate after Nth cycles at 1C rate, 45°C. The specific method is as follows: at 45°C, the formed battery was charged with 1C constant current/ constant voltage to 4.4V (LiNi_{0.5}Co_{0.2}Mn_{0.3}O₂/ artificial graphite battery)/ 4.2V (LiNi_{0.8}Co_{0.15}Al_{0.05}O₂/ SiC battery), the cutoff current was 0.01C, and then discharged to 3.0V with 1C constant current. After such charging/discharging for N cycles, the capacity retention after the Nth cycle was calculated to evaluate its high-temperature cycle performance.

The calculation formula of the Nth cycle capacity retention rate at 45°C 1C is as follows: 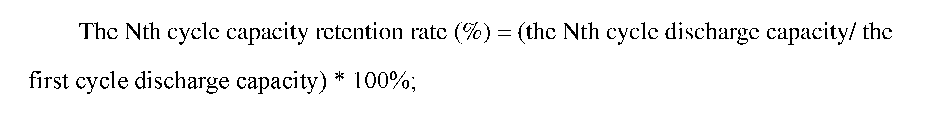 The Nth cycle capacity retention rate (%) = (the Nth cycle discharge capacity/ the first cycle discharge capacity) * 100%;

(2) Test method for capacity retention rate, capacity recovery rate and thickness expansion rate after 30 days of storage at 60°C: the formed battery was charged to 4.4V (LiNi_{0.5}Co_{0.2}Mn_{0.3}O₂/ artificial graphite battery)/ 4.2V (LiNi_{0.8}Co_{0.15}Al_{0.05}O₂/ Si-C battery) at room temperature with 1C constant current/ constant voltage, the cutoff current was 0.01C, then discharged to 3.0V with 1C constant current, the initial discharge capacity of the battery was measured, then charged to 4.4V with 1C constant current/ constant voltage, the cutoff current was 0.01C, and the initial thickness of the battery was measured. Then the battery was stored at 60°C for 30 days, measured the thickness of the battery, discharged it to 3.0V with 1C constant current, measured the capacity retention of the battery, then charged it to the operating voltage with 1C constant current/ constant voltage, the cutoff current was 0.01C, then discharged it to 3.0V with 1C constant current, then measured the recovery capacity. The calculation formulas for capacity retention rate and capacity recovery rate are as follows:$Battery capacity retention rate \left(%\right) = \left(retention capacity/ initial capacity\right) ∗ 100 % ;$$Battery capacity recovery rate \left(%\right) = \left(recovery capacity/ initial capacity\right) ∗ 100 % ;$ 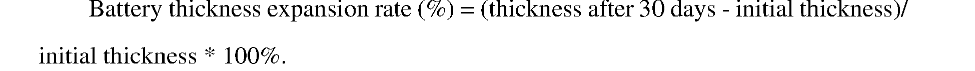 Battery thickness expansion rate (%) = (thickness after 30 days - initial thickness)/ initial thickness * 100%.

(3) Low-temperature discharge performance: at 25°C, the formed battery was charged to 4.4V with 1C constant current/constant voltage, then charged to drop the current to 0.01C with constant voltage, and then discharged to 3.0V with 1C constant current, the room temperature discharge capacity was recorded. Then charged to 4.4V with 1C constant current (LiNi_{0.5}Co_{0.2}Mn_{0.3}O₂ / artificial graphite battery) /4.2V (LiNi_{0.8}Co_{0.15}Al_{0.05}O₂ / silicon-carbon battery), then charged to drop the current to 0.01C with constant voltage, the battery was placed in an environment of -20°C for 12h, then discharge to 3.0V with 0.2C constant current, the discharge capacity of -20°C was recorded. 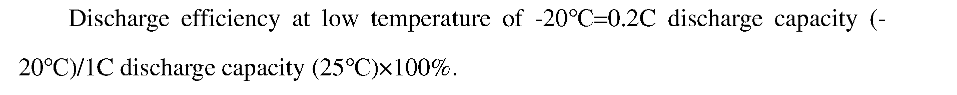 Discharge efficiency at low temperature of -20°C=0.2C discharge capacity (-20°C)/1C discharge capacity (25°C)×100%.

**Table 1**

| | Total weight percentage, composition and weight ratio of the solvent | Additive and weight percentage |
|---|---|---|
| Embodiment 1 | Total weight percentage: 87% FEC/PC/DEC=2/1/1 | Compound 1: 0.5% |
| Embodiment 2 | Total weight percentage: 85.5% FEC/PC/DEC=2/1/1 | Compound 1: 2% |
| Embodiment 3 | Total weight percentage: 85.5% FEC/PC/DEC=2/1/1 | Compound 2: 2% |
| Embodiment 4 | Total weight percentage: 83.5% FEC/PC/DEC=2/1/1 | Compound 1: 4% |
| Embodiment 5 | Total weight percentage: 85.5% DFEA/PC/DEC/EC=1/1/1/1 | Compound 1: 2% |
| Embodiment 6 | Total weight percentage: 85.5% DFEA/PC/DEC/EC=1/1/1/1 | Compound 2: 2% |
| Embodiment 7 | Total weight percentage: 84.5% DFEA/PC/DEC/EC=1/1/1/1 | Compound 1: 3% |
| Embodiment 8 | Total weight percentage: 85.5% FEC/PC/DFEA=2/1/1 | Compound 1: 2% |
| Embodiment 9 | Total weight percentage: 84.5% FEC/PC/DFEA=2/1/1 | Compound 1: 3% |
| Embodiment 10 | Total weight percentage: 85.5% FEC/PC/ Methyl 2,2,2-trifluoroethyl carbonate =2/1/1 | Compound 1: 2% |
| Embodiment 11 | Total weight percentage: 84.5% DFEA/PC/DEC/EC=1/1/1/1 | Compound 1: 2% |
| | | LiBOB: 1% |
| Embodiment 12 | Total weight percentage: 84.5% DFEA/PC/DEC/EC=1/1/1/1 | Compound 1: 2% |
| | | VC: 1% |
| Embodiment 13 | Total weight percentage: 84.5% DFEA/PC/DEC/EC=1/1/1/1 | Compound 1: 2% |
| | | PS: 1% |
| Comparative example 1 | Total weight percentage: 87.5% FEC/PC/DEC=2/1/1 | |
| Comparative example 2 | Total weight percentage: 87.5% DFEA/PC/DEC/EC=1/1/1/1 | |
| Comparative example 3 | Total weight percentage: 87.5% FEC/PC/DFEA=2/1/1 | |
| Comparative example 4 | Total weight percentage: 87.5% FEC/PC/ Methyl 2,2,2-trifluoroethyl carbonate =2/1/1 | |
| Comparative example 5 | Total weight percentage: 86.5% FEC/PC/DEC=2/1/1 | LiBOB: 1% |
| Comparative example 6 | Total weight percentage: 86.5% DFEA/PC/DEC/EC=1/1/1/1 | VC: 1% |
| Comparative example 7 | Total weight percentage: 86.5% DFEA/PC/DEC/EC=1/1/1/1 | PS: 1% |

The test results of Embodiments 1-13 and Comparative examples 1-7 are shown in Table 2 below.

**Table 2**

| | | | The 400th cycle capacity | After 30 days of storage at high temperature of 60°C | 0.2C Discharge |
|---|---|---|---|---|---|
| | retention rate at 45°C | Capacity retention rate | Capacity recovery rate | Thickness expansion rate | Efficiency (-20°C) |
| Embodiment 1 | 65.3% | 70.2% | 75.9% | 44.3% | 75.3% |
| Embodiment 2 | 76.9% | 80.6% | 85.1% | 24.9% | 75.0% |
| Embodiment 3 | 73.3% | 76.3% | 81.5% | 30.3% | 76.0% |
| Embodiment 4 | 85.1% | 85.4% | 90.5% | 17.8% | 75.6% |
| Embodiment 5 | 70.8% | 71.2% | 75.9% | 33.5% | 76.7% |
| Embodiment 6 | 68.5% | 68.3% | 74.1% | 34.6% | 74.2% |
| Embodiment 7 | 75.4% | 78.8% | 84.2% | 28.8% | 76.2% |
| Embodiment 8 | 76.9% | 78.4% | 84.1% | 20.4% | 76.0% |
| Embodiment 9 | 86.5% | 86.2% | 91.1% | 17.4% | 75.1% |
| Embodiment 10 | 73.2% | 77.7% | 83.2% | 30.5% | 73.8% |
| Embodiment 11 | 80.1% | 82.5% | 87.6% | 17.8% | 74.4% |
| Embodiment 12 | 78.5% | 79.3% | 84.2% | 30.1% | 73.7% |
| Embodiment 13 | 75.5% | 81.1% | 85.5% | 26.6% | 72.4% |
| Comparative example 1 | 51.4% | 45.5% | 52.1% | 52.4% | 75.1% |
| Comparative example 2 | 34.7% | 35.9% | 41.2% | 72.4% | 77.0% |
| Comparative example 3 | 54.5% | 51.5% | 55.3% | 65.4% | 75.3% |
| Comparative example 4 | 45.2% | 44.1% | 50.2% | 76.2% | 74.0% |
| Comparative example 5 | 65.4% | 63.5% | 66.6% | 48.5% | 71.4% |
| Comparative example 6 | 62.3% | 64.7% | 65.6% | 49.5% | 73.5% |
| Comparative example 7 | 55.8% | 66.5% | 66.6% | 50.5% | 72.2% |

Referring to Table 1, the Embodiments 1-4 and Comparative Example 1 are compared, in the lithium ion non-aqueous electrolyte of Embodiments 1-4 and Comparative Example 1, the additive composition and proportion of Compound A are the same (FEC/PC/DEC=2/1/1), however, the Comparative Example 1 was not added with Compound B, and Embodiments 1-4 were added with Compound B. The results showed that compared with Comparative Example 1 to which only compound A was added, the battery made of lithium ion non-aqueous electrolyte containing both compound A and compound B had obviously improved high-temperature cycle performance and high-temperature storage performance, reaching 85.1% (51.4% in Cited document 1) after 300 cycles at 45°C. The capacity retention rate, capacity recovery rate and thickness expansion rate after 30 days of storage at high temperature of 60°C, reached 85.4% (45.5% in Cited document 1), 90.5% (52.1% in Cited document 1), 17.8% (52.4% in Cited document 1), respectively, and the 0.2C Discharge Efficiency (-20°C) was 75.0-76%(75.1% in Cited document 1). It can be seen that the synergistic effect of the compound A and the compound B can obviously improve the high-temperature cycle performance and the high-temperature storage performance of the battery. Moreover, the combination of the compound A and the compound B has no obvious influence on the battery impedance, so that batteries made of the lithium ion non-aqueous electrolyte have better low-temperature performance. Meanwhile, the compound B is in the range of 0.5-4%, and the higher the percentage mass content is, the better the high-temperature cycle performance and high-temperature storage performance are.

Comparing the Embodiments 5-7 and Comparative Example 2, in the lithium ion non-aqueous electrolyte of Embodiments 5-7 and Comparative Example 2, the additive composition and proportion of Compound A are the same (DFEA/PC/DEC/EC=1/1/1/1), however, Comparative Example 2 was not added with Compound B, and Embodiments 5-7 were added with Compound B. The results showed that compared with Comparative Example 2 to which only compound A was added, the battery made of lithium ion non-aqueous electrolyte containing both compound A and compound B had obviously improved high-temperature cycle performance and high-temperature storage performance, reaching 75.4% (34.7% in Cited document 2) after 400 cycles at 45°C. The capacity retention rate, capacity recovery rate and thickness expansion rate after 30 days of storage at high temperature of 60°C, reached 78.8% (35.9% in Cited document 2), 84.2% (41.2% in Cited document 2), 28.8% (72.4% in Cited document 2), respectively, and the 0.2C Discharge Efficiency (-20°C) was 74.2-76.7% (77% in Cited document 2). It can be seen that the synergistic effect of the compound A and the compound B can obviously improve the high-temperature cycle performance and the high-temperature storage performance of the battery. Meanwhile, the combination of the compound A and the compound B has no obvious influence on the battery impedance, so that batteries made of the lithium ion non-aqueous electrolyte have better low-temperature performance.

Comparing the Embodiments 8-9 and Comparative Example 3, in the lithium ion non-aqueous electrolyte of Embodiments 8-9 and Comparative Example 3, the additive composition and proportion of Compound A are the same ( FEC/PC/DFEA=2/1/1, however, Comparative Example 3 was not added with Compound B, and Embodiments 8-9 were added with Compound B. The results showed that compared with Comparative Example 3 to which only compound A was added, the battery made of lithium ion non-aqueous electrolyte containing both compound A and compound B had obviously improved high-temperature cycle performance and high-temperature storage performance, reaching 86.5% (54.5% in Cited document 3) after 400 cycles at 45°C. The capacity retention rate, capacity recovery rate and thickness expansion rate after 30 days of storage at high temperature of 60°C, reached 86.2% (51.5% in Cited document 3), 91.1% (55.3% in Cited document 3), 17.4% (65.4% in Cited document 3), respectively, and the 0.2C Discharge Efficiency (-20°C) was 74.4-75.1% (75.3% in Cited document 3). It can be seen that the synergistic effect of the compound A and the compound B can obviously improve the high-temperature cycle performance and the high-temperature storage performance of the battery. Meanwhile, the combination of the compound A and the compound B has no obvious influence on the battery impedance, so that batteries made of the lithium ion non-aqueous electrolyte have better low-temperature performance.

Comparing the Embodiment 10 and Comparative Example 4, in the lithium ion non-aqueous electrolyte of Embodiment 10 and Comparative Example 4, the additive composition and proportion of Compound A are the same FEC/PC/ Methyl 2,2,2-trifluoroethyl carbonate =2/1/1), however, Comparative Example 4 was not added with Compound B, and Embodiment 10 was added with Compound B. The results showed that compared with Comparative Example 4 to which only compound A was added, the battery made of lithium ion non-aqueous electrolyte containing both compound A and compound B had obviously improved high-temperature cycle performance and high-temperature storage performance, reaching 73.2% (45.2% in Cited document 4) after 400 cycles at 45°C. The capacity retention rate, capacity recovery rate and thickness expansion rate after 30 days of storage at high temperature of 60°C, reached 77.7% (44.1% in Cited document 4), 83.2% (50.2% in Cited document 4), 30.5% (76.2% in Cited document 4), respectively, and the 0.2C Discharge Efficiency (-20°C) was 73.8% (74% in Cited document 4). It can be seen that the synergistic effect of the compound A and the compound B can obviously improve the high-temperature cycle performance and the high-temperature storage performance of the battery. Meanwhile, the combination of the compound A and the compound B has no obvious influence on the battery impedance, so that batteries made of the lithium ion non-aqueous electrolyte have better low-temperature performance.

Comparing the Embodiments 11-13 and Comparative Example 5-7, in the lithium ion non-aqueous electrolyte of Embodiments 11-13 and Comparative Example 5-7, the additive composition and proportion of Compound A are the same (DFEA/PC/DEC/EC=1/1/1/1), moreover, they were added with the same amount of LiBOB, VC and PS. However, the Comparative Examples 5-7 were not added with Compound B, and Embodiments 11-13 were added with Compound B. The results showed that, compared with Comparative Examples 5-7, the battery made of lithium ion non-aqueous electrolyte of Embodiment 11-13 had obviously improved high-temperature cycle performance and high-temperature storage performance, reaching 80.1% (65.4% in Cited document 5) after 400 cycles at 45°C. The capacity retention rate, capacity recovery rate and thickness expansion rate after 30 days of storage at high temperature of 60°C, reached 82.5% (63.5% in Cited document 5), 87.6% (66.6% in Cited document 5), 17.8% (48.5% in Cited document 5), respectively, and the 0.2C Discharge Efficiency (-20°C) was 74.4% (73.4% in Cited document 5). It can be seen that the synergistic effect of the compound A and the compound B can obviously improve the high-temperature cycle performance and the high-temperature storage performance of the battery. Meanwhile, the combination of the compound A and the compound B has no obvious influence on the battery impedance, so that batteries made of the lithium ion non-aqueous electrolyte have better low-temperature performance.

**Table 3**

| | Total weight percentage, composition and weight ratio of the solvent | Additive and weight percentage |
|---|---|---|
| Embodiment 14 | Total weight percentage: 85.5% FEC/PC/DEC=2/1/1 | Compound 1: 2% |
| Embodiment 15 | Total weight percentage: 85.5% DFEA/PC/DEC/EC=1/1/1/1 | Compound 1: 2% |
| Embodiment 16 | Total weight percentage: 85.5% FEC/PC/DFEA=2/1/1 | Compound 1: 2% |
| Embodiment 17 | Total weight percentage: 85.5% FEC/PC/ Methyl 2,2,2-trifluoroethyl carbonate =2/1/1 | Compound 1: 2% |
| Comparative example 8 | Total weight percentage: 87.5% FEC/PC/DEC=2/1/1 | |
| Comparative example 9 | Total weight percentage: 87.5% DFEA/PC/DEC/EC=1/1/1/1 | |
| Comparative example 10 | Total weight percentage: 87.5% FEC/PC/DFEA=2/1/1 | |
| Comparative example 11 | Total weight percentage: 87.5% FEC/PC/ Methyl 2,2,2-trifluoroethyl carbonate =2/1/1 | |

The test results of Embodiments 14-17 and Comparative examples 8-11 are shown in Table 4 below.

**Table 4**

| | | | The 200th cycle capacity | After 30 days of storage at high temperature of 60°C | 0.2C Discharge |
|---|---|---|---|---|---|
| | retention rate at 45°C | Capacity retention rate | Capacity recovery rate | Thickness expansion rate | Efficiency (-20°C) |
| Embodiment 14 | 82.3% | 75.2% | 80.9% | 31.3% | 78.3% |
| Embodiment 15 | 71.9% | 65.6% | 70.1% | 35.9% | 78.0% |
| Embodiment 16 | 84.3% | 75.3% | 80.5% | 30.3% | 79.0% |
| Embodiment 17 | 76.1% | 70.4% | 75.5% | 32.8% | 78.6% |
| Comparative example 8 | 62.4% | 50.5% | 55.1% | 43.4% | 78.1% |
| Comparative example 9 | 54.8% | 40.9% | 45.2% | 64.4% | 77.0% |
| Comparative example 10 | 64.3% | 55.5% | 60.3% | 54.4% | 77.3% |
| Comparative example 11 | 60.4% | 49.1% | 55.2% | 65.2% | 76.0% |

Comparing the Embodiments 14-17 and Comparative Example 8-11, in the lithium ion non-aqueous electrolyte of Embodiments 14-17 and Comparative Example 8-11, the addictive compositions of compound A were FEC/PC/DEC=2/1/1, DFEA/PC/DEC/EC=1/1/1/1, FEC/PC/DFEA=2/1/1, FEC/PC/methyl 2,2,2-trifluoroethyl carbonate=2/1/1, however, Comparative Examples 8-11 were not added with Compound B, and Embodiments 14-17 were added with Compound B. The results showed that compared with Comparative Examples 8-11 to which only compound A was added, the battery made of lithium ion non-aqueous electrolyte containing both compound A and compound B had obviously improved high-temperature cycle performance and high-temperature storage performance, reaching 84.3% (64.3% in Cited document 10) after 200 cycles at 45°C. The capacity retention rate, capacity recovery rate and thickness expansion rate after 14 days of storage at high temperature of 60°C, reached 75.3% (55.5% in Cited document 10), 80.5% (60.3% in Cited document 10), 30.3% (54.4% in Cited document 10), respectively, and the 0.2C Discharge Efficiency (-20°C) was 78.0-79% (77.3% in Cited document 10). It can be seen that the synergistic effect of the compound A and the compound B can obviously improve the high-temperature cycle performance and the high-temperature storage performance of the battery. Moreover, the combination of the compound A and the compound B has no obvious influence on the battery impedance, so that batteries made of the lithium ion non-aqueous electrolyte have better low-temperature performance.

## Claims

1. A non-aqueous electrolyte for lithium ion battery, comprising a compound A and a compound B, wherein the compound A is at least one of compounds represented by the following structural formula I and formula II; the compound B is a compound represented by the following structural formula IV,
formula I: R₁-COO-R₂;
formula II: R₃-OCOO-R₄; 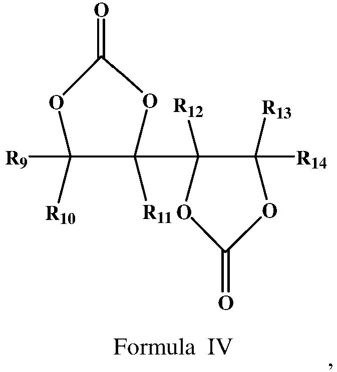
in formula I, R₁ is a hydrogen atom, a C1-C5 hydrocarbon group or a C1-C5 fluorohydrocarbon group, R₂ is a C1-C5 hydrocarbon group or a C1-C5 fluorohydrocarbon group, and at least one of R₁ and R₂ contains a fluorine atom;
in formula II, R₃ and R₄ are each independently selected from C1-C5 hydrocarbon group or C1-C5 fluorohydrocarbon group, and at least one of R₃ and R₄ contains a fluorine atom;
in formula IV, R₉, R₁₀, R₁₁, R₁₂, R₁₃ and R₁₄ are each independently selected from one of hydrogen atom, fluorine atom or C1-C5 group.

2. The non-aqueous electrolyte for lithium ion battery of claim 1, wherein in formula IV, the C1-C5 group is selected from a hydrocarbon group, fluorinated hydrocarbon group, oxygen-containing hydrocarbon group, silicon-containing hydrocarbon group, and cyano-substituted hydrocarbon group.

3. The non-aqueous electrolyte for lithium ion battery of claim 1, wherein in formula IV, R₉, R₁₀, R₁₁, R₁₂, R₁₃ and R₁₄ are each independently selected from a hydrogen atom, fluorine atom, methyl group, ethyl group, methoxyl group, ethyoxyl group, trimethylsiloxy group, cyano group or trifluoromethyl group.

4. The non-aqueous electrolyte for lithium ion battery of claim 1, wherein the compound B comprises one or more of compounds 1-9 represented by the following structural formulae,

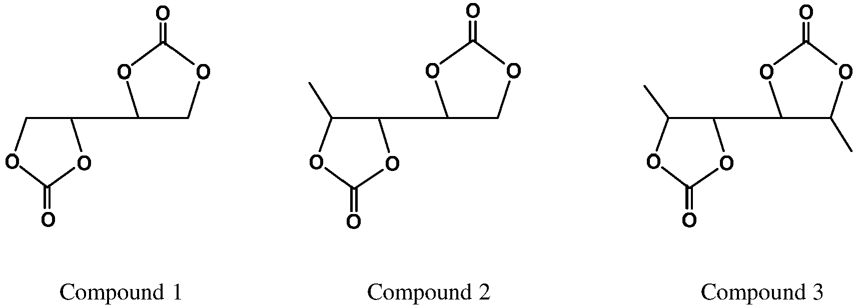

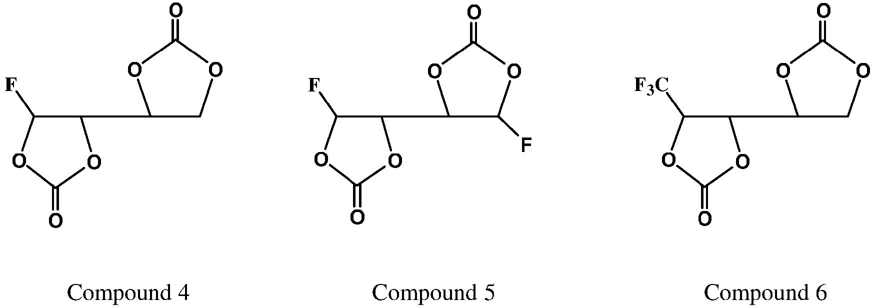

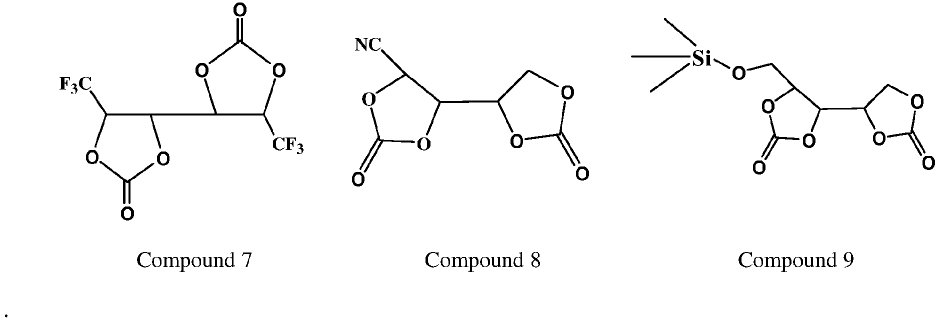

5. The non-aqueous electrolyte for lithium ion battery of any one of claims 1-4, wherein the percentage mass content of the compound B is 0.1-5% based on the total mass of the non-aqueous electrolyte for lithium ion battery being 100%.

6. The non-aqueous electrolyte for lithium ion battery of claim 1, wherein, the compound represented by formula I is selected from one or more of H₃CCOOCH₂CF₂H, H₃CH₂CCOOCH₂CF₂H, HF₂CH₂CCOOCH₃, HF₂CH₂CCOOCH₂CH₃, HF₂CH₂CH₂CCOOCH₂CH₃, H₃CCOOCH₂CH₂CF₂H, H₃CH₂CCOOCH₂CH₂CF₂H, CH₃COOCH₂CF₃, HCOOCH₂CHF₂, HCOOCH₂CF₃ and CH₃COOCH₂CF₂CF₂H.

7. The non-aqueous electrolyte for lithium ion battery of claim 1, wherein the compound represented by formula II is selected from one or more of CH₃OCOOCH₂CF₂H, CH₃OCOOCH₂CF₃, CH₃OCOOCH₂CF₂CF₂H, HCF₂CH₂OCOOCH₂CH₃ and CF₃CH₂OCOOCH₂CH₃.

8. The non-aqueous electrolyte for lithium ion battery of claim 1, wherein the compound A further comprises a compound represented by formula III, 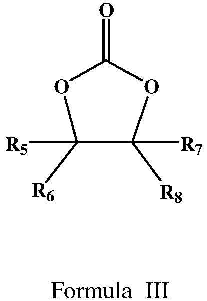 in formula III, R₅, R₆, R₇ and R₈ are each independently selected from hydrogen atom, fluorine atom, C1-C4 hydrocarbon group or C1-C4 fluorohydrocarbon group, and at least one of R₅, R₆, R₇ and R₈ contains a fluorine atom.

9. The non-aqueous electrolyte for lithium ion battery of claim 8, wherein the compound represented by formula III is selected from one or more of compounds 10-13 represented by the following structures,

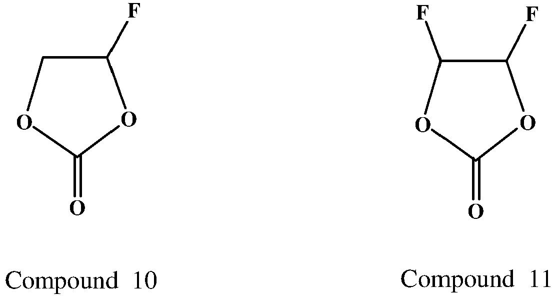

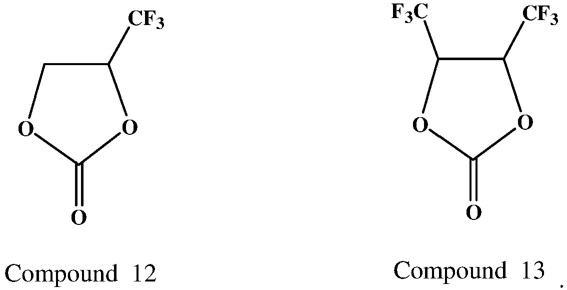

10. The non-aqueous electrolyte for lithium ion battery of claim 1, wherein the percentage mass content of the compound A is greater than 5% and less than 80% based on the total mass of the non-aqueous electrolyte for lithium ion battery being 100%.

11. The non-aqueous electrolyte for lithium ion battery of claim 8, wherein R₅ in formula III is fluorine, R₆, R₇ and R₈ are each independently selected from hydrogen atom, fluorine atom, C1-C4 hydrocarbon group or C1-C4 fluorohydrocarbon group, and R₆, R₇ and R₈ cannot be hydrogen atom at the same time; or R₅ is C1-C4 fluorine-containing hydrocarbon group, R₆, R₇ and R₈ are independently selected from hydrogen atom, fluorine atom, C1-C4 hydrocarbon group or C1-C4 fluorine-containing hydrocarbon group.

12. The non-aqueous electrolyte for lithium ion battery of claim 1, wherein the lithium ion non-aqueous electrolyte further comprises at least one of unsaturated cyclic carbonate compounds and sultone compounds.

13. The non-aqueous electrolyte for lithium ion battery of claim 12, wherein the unsaturated cyclic carbonate compound comprises at least one of vinylene carbonate and vinyl ethylene carbonate;
the sultone compound is selected from at least one of 1,3- propane sultone, 1,4-butane sultone, and 1,3-propene sultone;
the percentage mass content of the unsaturated cyclic carbonate compound is 0.1-5% based on the total mass of the non-aqueous electrolyte of the lithium ion battery being 100%; the percentage mass content of the sultone compound is 0.1-5% based on the total mass of the non-aqueous electrolyte of the lithium ion battery being 100%.

14. A non-aqueous electrolyte for lithium ion battery, comprising a compound A and a compound B, wherein the compound A is a compound represented by the following structural formula III; the compound B is a compound represented by the following structural formula IV, 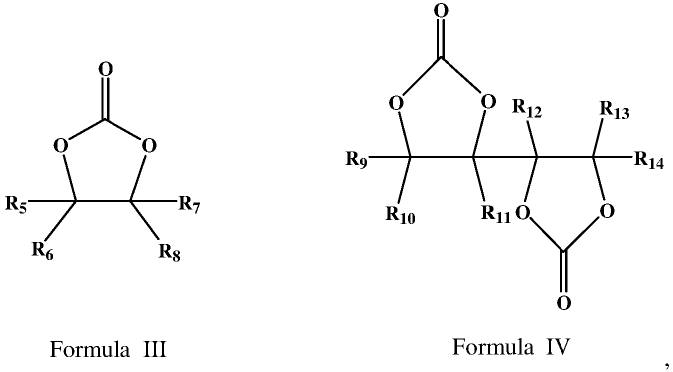
in formula III, R₅ is fluorine, R₆, R₇ and R₈ are each independently selected from hydrogen atom, fluorine atom, C1-C4 hydrocarbon group or C1-C4 fluorohydrocarbon group, and R₆, R₇ and R₈ cannot be hydrogen atom at the same time; or R₅ is a C1-C4 fluoroalkyl group, R₆, R₇ and R₈ are independently selected from hydrogen atom, fluorine atom, C1-C4 hydrocarbyl group, or C1-C4 fluoroalkyl group;
in formula IV, R₉, R₁₀, R₁₁, R₁₂, R₁₃ and R₁₄ are each independently selected from one of hydrogen atom, fluorine atom or C1-C5 group.

15. The non-aqueous electrolyte for lithium ion battery of claim 14, wherein the compound represented by formula III is selected from one or more of compounds 11-13 represented by the following structures, 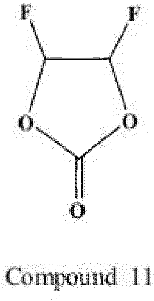 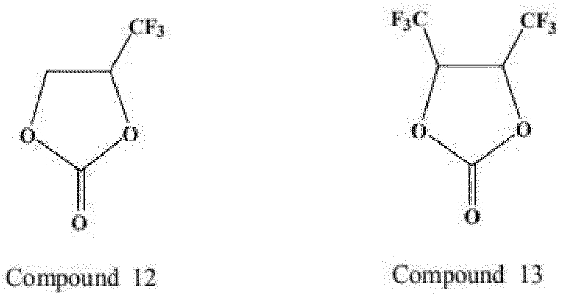 the compound B comprises one or more of compounds 1-9 represented by the following structural formulae,

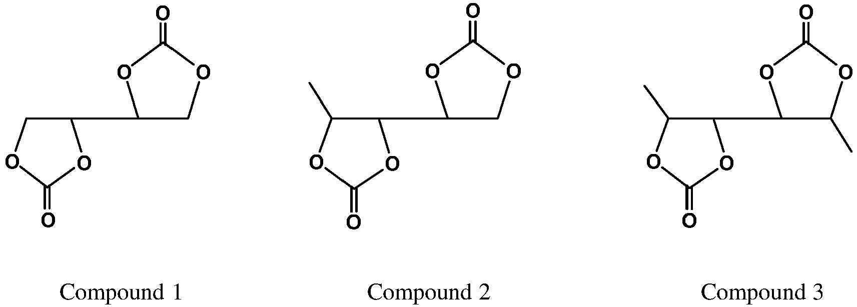

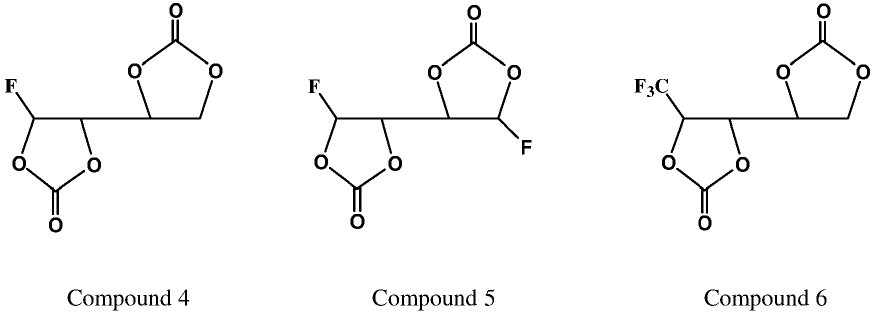

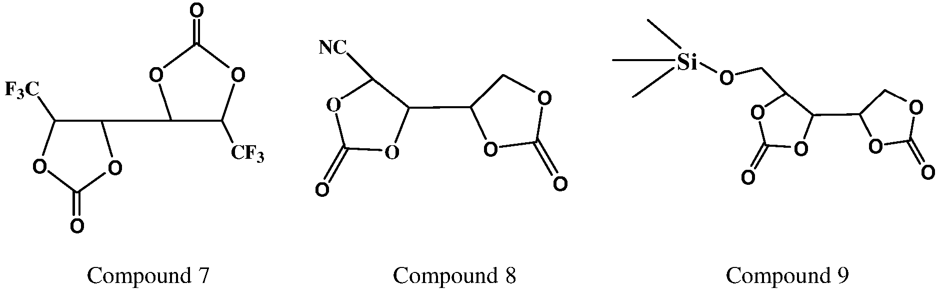

16. The non-aqueous electrolyte for lithium ion battery of claim 14, wherein the percentage mass content of the compound B is 0.1-5% based on the total mass of the non-aqueous electrolyte for lithium ion battery being 100%;
the percentage mass content of the compound A is greater than 5% and less than or equal to 80% based on the total mass of the non-aqueous electrolyte for lithium ion battery being 100%.

17. The non-aqueous electrolyte for lithium ion battery of claim 14, wherein the lithium ion non-aqueous electrolyte further comprises at least one of unsaturated cyclic carbonate compounds and sultone compounds;
the percentage mass content of the unsaturated cyclic carbonate compound is 0.1-5% based on the total mass of the non-aqueous electrolyte of the lithium ion battery being 100%; the percentage mass content of the sultone compound is 0.1-5% based on the total mass of the non-aqueous electrolyte of the lithium ion battery being 100%.

18. A lithium ion battery, comprising a positive electrode, a negative electrode, a separator interposed between the positive electrode and the negative electrode, and an electrolyte, wherein the electrolyte is the non-aqueous electrolyte for lithium ion battery of any of claims 1-17.

19. The lithium ion battery of claim 18, wherein the positive electrode comprises a positive electrode active material, and the positive electrode active material is at least one of LiNiₓCo_{y}Mn_{z}L_{(1-x-y-z)}O₂, LiCo_{x'}L_{(1-x')}O₂, LiNi_{x"}L'_{y'}Mn_{(2-x"-y' )} O₄ and Li_{z'}MPO₄, wherein, L is at least one of Al, Sr, Mg, Ti, Ca, Zr, Zn, Si or Fe, 0≤x≤1, 0≤y≤1, 0≤z≤1, 0<x+y+z≤1, 0<x'≤1, 0.3≤x"≤0.6, 0.01≤y'≤0.2, L' is at least one of Co, Al, Sr, Mg, Ti, Ca, Zr, Zn, Si and Fe; 0.5≤z'≤1, M is at least one of Fe, Mn and Co.

## Patentansprüche

1. Ein wasserfreier Elektrolyt für eine Lithium-Ionen-Batterie, umfassend eine Verbindung A und eine Verbindung B, wobei die Verbindung A mindestens eine der durch die folgenden Strukturformeln I und II dargestellten Verbindungen ist; die Verbindung B eine durch die folgende Strukturformel IV dargestellte Verbindung ist,
Formel I: R₁-COO-R₂;
Formel II: R₃-OCOO-R₄; 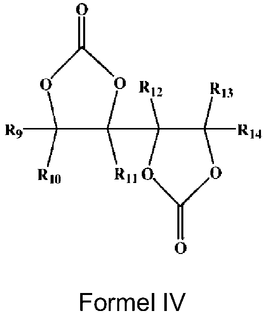
wobei in Formel I R₁ ein Wasserstoffatom, eine C1-C5-Kohlenwasserstoffgruppe oder eine C1-C5-Fluorkohlenwasserstoffgruppe ist, R₂ eine C1-C5-Kohlenwasserstoffgruppe oder eine C1-C5-Fluorkohlenwasserstoffgruppe ist und mindestens eines von R₁ und R₂ ein Fluoratom enthält;
wobei in Formel II R₃ und R₄ jeweils unabhängig voneinander aus einer C1-C5-Kohlenwasserstoffgruppe oder einer C1-C5-Fluorkohlenwasserstoffgruppe ausgewählt sind und mindestens eines von R₃ und R₄ ein Fluoratom enthält;
wobei in Formel IV R₉, R₁₀, R₁₁, R₁₂, R₁₃ und R₁₄ jeweils unabhängig voneinander aus einem Wasserstoffatom, einem Fluoratom oder einer C1-C5-Gruppe ausgewählt sind.

2. Der wasserfreie Elektrolyt für eine Lithium-Ionen-Batterie gemäß Anspruch 1, wobei in Formel IV die C1-C5-Gruppe ausgewählt ist aus einer Kohlenwasserstoffgruppe, einer fluorierten Kohlenwasserstoffgruppe, einer sauerstoffhaltigen Kohlenwasserstoffgruppe, einer siliciumhaltigen Kohlenwasserstoffgruppe und einer cyanosubstituierten Kohlenwasserstoffgruppe.

3. Der wasserfreie Elektrolyt für eine Lithium-Ionen-Batterie gemäß Anspruch 1, wobei in Formel IV R₉, R₁₀, R₁₁, R₁₂, R₁₃ und R₁₄ jeweils unabhängig voneinander ausgewählt sind aus einem Wasserstoffatom, einem Fluoratom, einer Methylgruppe, einer Ethylgruppe, einer Methoxygruppe, einer Ethoxygruppe, einer Trimethylsiloxygruppe, einer Cyanogruppe oder einer Trifluormethylgruppe.

4. Der wasserfreie Elektrolyt für eine Lithium-Ionen-Batterie gemäß Anspruch 1, wobei die Verbindung B eine oder mehrere der durch die folgenden Strukturformeln dargestellten Verbindungen 1 bis 9 umfasst,

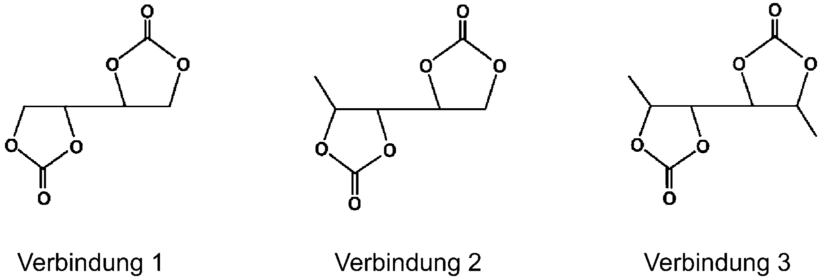

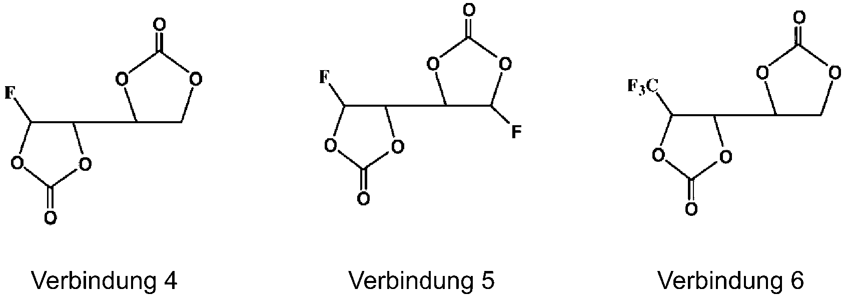

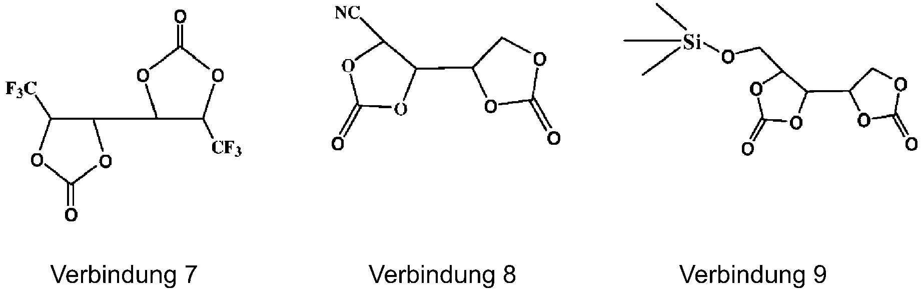

5. Der wasserfreie Elektrolyt für eine Lithium-Ionen-Batterie gemäß einem der Ansprüche 1 bis 4, wobei der Massenanteil der Verbindung B 0,1 bis 5 % beträgt, bezogen auf die Gesamtmasse des wasserfreien Elektrolyten für eine Lithium-Ionen-Batterie von 100 %.

6. Der wasserfreie Elektrolyt für eine Lithium-Ionen-Batterie gemäß Anspruch 1, wobei die durch Formel I dargestellte Verbindung aus einer oder mehreren von H₃CCOOCH₂CF₂H, H₃CH₂CCOOCH₂CF₂H, HF₂CH₂CCOOCH₃, HF₂CH₂CCOOCH₂CH₃, HF₂CH₂CH₂CCOOCH₂CH₃, H₃CCOOCH₂CH₂CF₂H, H₃CH₂CCOOCH₂CH₂CF₂H, CH₃COOCH₂CF₃, HCOOCH₂CHF₂, HCOOCH₂CF₃ und CH₃COOCH₂CF₂CF₂H. ausgewählt ist.

7. Der wasserfreie Elektrolyt für eine Lithium-Ionen-Batterie gemäß Anspruch 1, wobei die durch Formel II dargestellte Verbindung aus einer oder mehreren von CH₃OCOOCH₂CF₂H, CH₃OCOOCH₂CF₃, CH₃OCOOCH₂CF₂CF₂H, HCF₂CH₂OCOOCH₂CH₃ und CF₃CH₂OCOOCH₂CH₃ ausgewählt ist.

8. Der wasserfreie Elektrolyt für eine Lithium-Ionen-Batterie gemäß Anspruch 1, wobei die Verbindung A ferner eine durch Formel III dargestellte Verbindung umfasst. 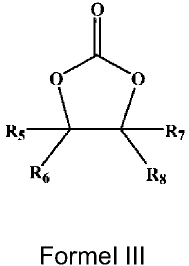 wobei in Formel III R₅, R₆, R₇ und R₈ jeweils unabhängig voneinander ausgewählt sind aus einem Wasserstoffatom, einem Fluoratom, einer C1-C4-Kohlenwasserstoffgruppe oder einer C1-C4-Fluorkohlenwasserstoffgruppe und mindestens eines von R₅, R₆, R₇ und R₈ ein Fluoratom enthält.

9. Der wasserfreie Elektrolyt für eine Lithium-Ionen-Batterie gemäß Anspruch 8, wobei die durch Formel III dargestellte Verbindung aus einer oder mehreren der durch die folgenden Strukturen dargestellten Verbindungen 10 bis 13 ausgewählt ist,

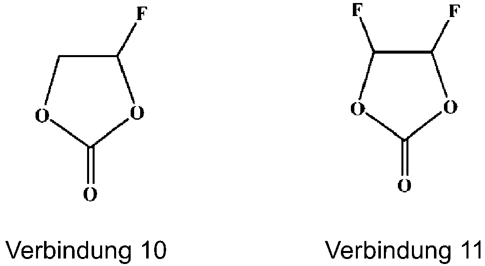

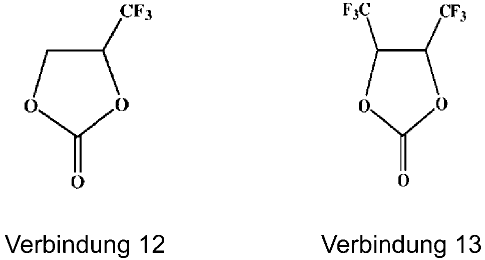

10. Der wasserfreie Elektrolyt für eine Lithium-Ionen-Batterie gemäß Anspruch 1, wobei der Massenanteil der Verbindung A größer als 5 % und kleiner als 80 % ist, bezogen auf die Gesamtmasse des wasserfreien Elektrolyten für eine Lithium-Ionen-Batterie von 100 %.

11. Der wasserfreie Elektrolyt für eine Lithium-Ionen-Batterie gemäß Anspruch 8, wobei R₅ in Formel III Fluor ist, R₆, R₇ und R₈ jeweils unabhängig voneinander ausgewählt sind aus einem Wasserstoffatom, einem Fluoratom, einer C1-C4-Kohlenwasserstoffgruppe oder einer C1-C4-Fluorkohlenwasserstoffgruppe und R₆, R₇ und R₈ nicht gleichzeitig Wasserstoffatome sein können; oder wobei R₅ eine C1-C4-fluorhaltige Kohlenwasserstoffgruppe ist und R₆, R₇ und R₈ unabhängig voneinander ausgewählt sind aus einem Wasserstoffatom, einem Fluoratom, einer C1-C4-Kohlenwasserstoffgruppe oder einer C1-C4-fluorhaltigen Kohlenwasserstoffgruppe.

12. Der wasserfreie Elektrolyt für eine Lithium-Ionen-Batterie gemäß Anspruch 1, wobei der wasserfreie Lithium-Ionen-Elektrolyt ferner mindestens eine Verbindung ausgewählt aus ungesättigten cyclischen Carbonatverbindungen und Sultonverbindungen umfasst.

13. Der wasserfreie Elektrolyt für eine Lithium-Ionen-Batterie gemäß Anspruch 12, wobei die ungesättigte cyclische Carbonatverbindung mindestens eine von Vinylencarbonat und Vinyl-ethylencarbonat umfasst;
wobei die Sultonverbindung aus mindestens einer von 1,3-Propansulton, 1,4-Butansulton und 1,3-Propensulton ausgewählt ist;
wobei der Massenanteil der ungesättigten cyclischen Carbonatverbindung 0,1 bis 5 % beträgt, bezogen auf die Gesamtmasse des wasserfreien Elektrolyten der Lithium-Ionen-Batterie von 100 %; wobei der Massenanteil der Sultonverbindung 0,1 bis 5 % beträgt, bezogen auf die Gesamtmasse des wasserfreien Elektrolyten der Lithium-Ionen-Batterie von 100 %.

14. Ein wasserfreier Elektrolyt für eine Lithium-Ionen-Batterie, umfassend eine Verbindung A und eine Verbindung B, wobei die Verbindung A eine durch die folgende Strukturformel III dargestellte Verbindung ist; die Verbindung B eine durch die folgende Strukturformel IV dargestellte Verbindung ist, 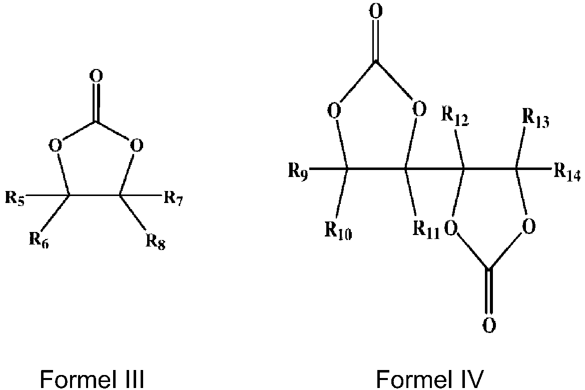
wobei in Formel III R₅ Fluor ist, R₆, R₇ und R₈ jeweils unabhängig voneinander ausgewählt sind aus einem Wasserstoffatom, einem Fluoratom, einer C1-C4-Kohlenwasserstoffgruppe oder einer C1-C4-Fluorkohlenwasserstoffgruppe und R₆, R₇ und R₈ nicht gleichzeitig Wasserstoffatome sein können; oder wobei R₅ eine C1-C4-Fluoralkylgruppe ist und R₆, R₇ und R₈ unabhängig voneinander ausgewählt sind aus einem Wasserstoffatom, einem Fluoratom, einem C1-C4-Kohlenwasserstoffrest oder einem C1-C4-Fluoralkylrest;
wobei in Formel IV R₉, R₁₀, R₁₁, R₁₂, R₁₃ und R₁₄ jeweils unabhängig voneinander aus einem Wasserstoffatom, einem Fluoratom oder einer C1-C5-Gruppe ausgewählt sind.

15. Der wasserfreie Elektrolyt für eine Lithium-Ionen-Batterie gemäß Anspruch 14, wobei die durch Formel III dargestellte Verbindung aus einer oder mehreren der durch die folgenden Strukturen dargestellten Verbindungen 11 bis 13 ausgewählt ist, 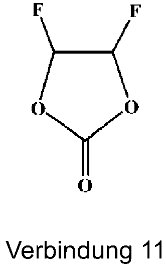 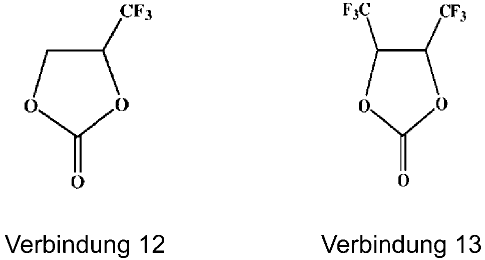 wobei die Verbindung B eine oder mehrere der durch die folgenden Strukturformeln dargestellten Verbindungen 1 bis 9 umfasst,

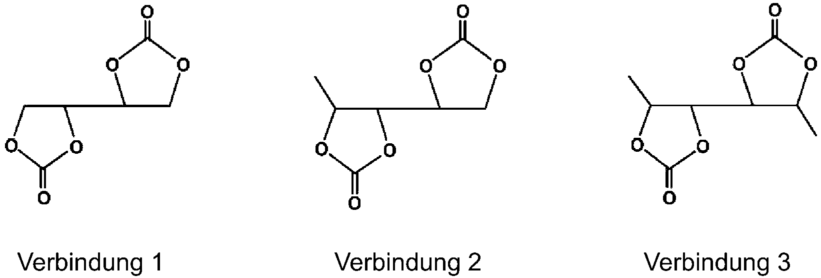

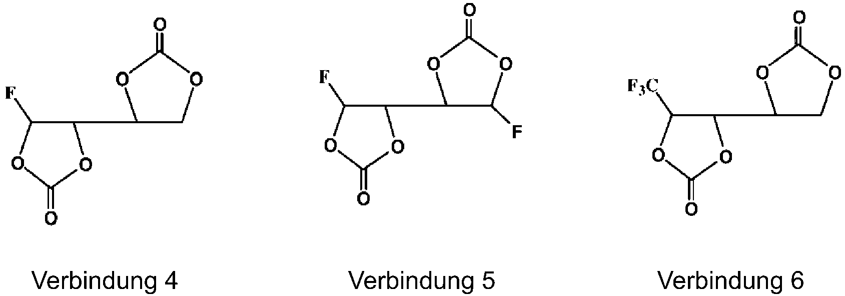

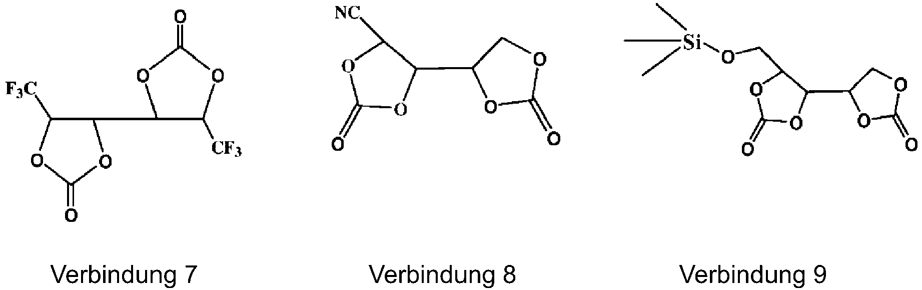

16. Der wasserfreie Elektrolyt für eine Lithium-Ionen-Batterie gemäß Anspruch 14, wobei der Massenanteil der Verbindung B 0,1 bis 5 % beträgt, bezogen auf die Gesamtmasse des wasserfreien Elektrolyten für eine Lithium-Ionen-Batterie von 100 %;
wobei der Massenanteil der Verbindung A größer als 5 % und kleiner oder gleich 80 % ist, bezogen auf die Gesamtmasse des wasserfreien Elektrolyten für eine Lithium-Ionen-Batterie von 100 %.

17. Der wasserfreie Elektrolyt für eine Lithium-Ionen-Batterie gemäß Anspruch 14, wobei der wasserfreie Lithium-Ionen-Elektrolyt ferner mindestens eine Verbindung aus ungesättigten cyclischen Carbonatverbindungen und Sultonverbindungen umfasst;
wobei der Massenanteil der ungesättigten cyclischen Carbonatverbindung 0,1 bis 5 % beträgt, bezogen auf die Gesamtmasse des wasserfreien Elektrolyten der Lithium-Ionen-Batterie von 100 %; wobei der Massenanteil der Sultonverbindung 0,1 bis 5 % beträgt, bezogen auf die Gesamtmasse des wasserfreien Elektrolyten der Lithium-Ionen-Batterie von 100 %.

18. Eine Lithium-Ionen-Batterie, umfassend eine positive Elektrode, eine negative Elektrode, einen zwischen der positiven Elektrode und der negativen Elektrode angeordneten Separator und einen Elektrolyten, wobei der Elektrolyt der wasserfreie Elektrolyt für eine Lithium-Ionen-Batterie gemäß einem der Ansprüche 1 bis 17 ist.

19. Die Lithium-Ionen-Batterie gemäß Anspruch 18, wobei die positive Elektrode ein positives Elektrodenaktivmaterial umfasst und das positive Elektrodenaktivmaterial mindestens eines von LiNiₓCo_{y}Mn_{z}L_{(1-x-y-z)}O₂, LiCo_{x'}L_{(1-x')}O₂, LiNi_{x"}L'_{y}'Mn_{(2-x"-y')}O₄ und Li_{z'}MPO₄ ist, wobei L mindestens eines von Al, Sr, Mg, Ti, Ca, Zr, Zn, Si oder Fe ist, 0≤x≤1, 0≤y≤1, 0≤z≤1, 0<x+y+z≤1, 0<x'≤1, 0.3≤x"≤0.6, 0.01≤y'≤0.2, L' mindestens eines von Co, Al, Sr, Mg, Ti, Ca, Zr, Zn, Si und Fe ist; 0.5≤z'≤1, wobei M mindestens eines von Fe, Mn und Co ist.

## Revendications

1. Un électrolyte non aqueux pour une batterie au lithium-ion, comprenant un composé A et un composé B, dans lequel le composé A est au moins l'un des composés représentés par les formules structurales I et II suivantes ; le composé B est un composé représenté par la formule structurale IV suivante,
formule I : R₁-COO-R₂ ;
formule II : R₃-OCOO-R₄ ; 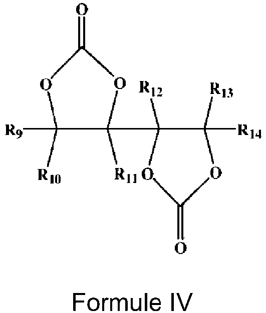
dans la formule I, R₁ est un atome d'hydrogène, un groupe hydrocarboné en C1-C5 ou un groupe fluorohydrocarboné en C1-C5, R₂ est un groupe hydrocarboné en C1-C5 ou un groupe fluorohydrocarboné en C1-C5, et au moins l'un de R₁ et R₂ contient un atome de fluor ;
dans la formule II, R₃ et R₄ sont chacun indépendamment choisis parmi un groupe hydrocarboné en C1-C5 ou un groupe fluorohydrocarboné en C1-C5, et au moins l'un de R₃ et R₄ contient un atome de fluor ;
dans la formule IV, R₉, R₁₀, R₁₁, R₁₂, R₁₃ et R₁₄ sont chacun indépendamment choisis parmi un atome d'hydrogène, un atome de fluor ou un groupe en C1-C5.

2. L'électrolyte non aqueux pour une batterie au lithium-ion selon la revendication 1, dans lequel, dans la formule IV, le groupe en C1-C5 est choisi parmi un groupe hydrocarboné, un groupe hydrocarboné fluoré, un groupe hydrocarboné contenant de l'oxygène, un groupe hydrocarboné contenant du silicium et un groupe hydrocarboné substitué par un groupe cyano.

3. L'électrolyte non aqueux pour une batterie au lithium-ion selon la revendication 1, dans lequel, dans la formule IV, R₉, R₁₀, R₁₁, R₁₂, R₁₃ et R₁₄ sont chacun indépendamment choisis parmi un atome d'hydrogène, un atome de fluor, un groupe méthyle, un groupe éthyle, un groupe méthoxyle, un groupe éthoxyle, un groupe triméthylsiloxy, un groupe cyano ou un groupe trifluorométhyle.

4. L'électrolyte non aqueux pour une batterie au lithium-ion selon la revendication 1, dans lequel le composé B comprend un ou plusieurs des composés 1 à 9 représentés par les formules structurales suivantes,

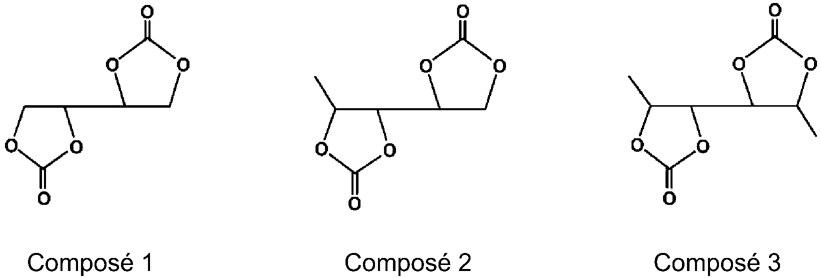

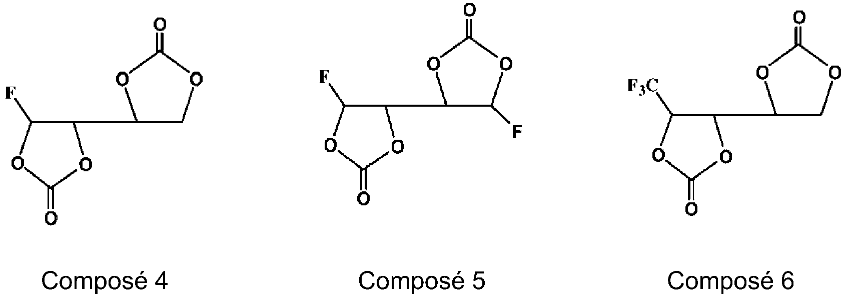

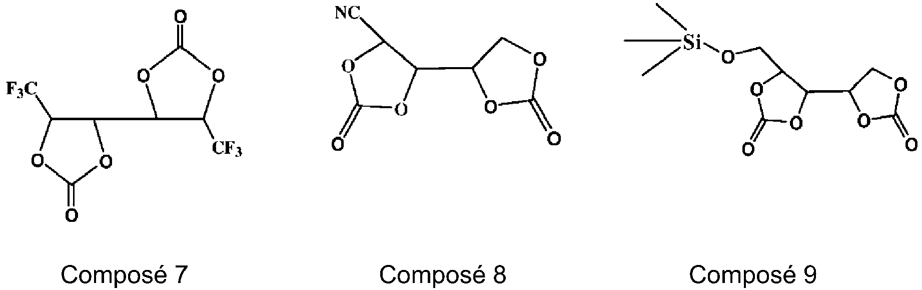

5. L'électrolyte non aqueux pour une batterie au lithium-ion selon l'une quelconque des revendications 1 à 4, dans lequel le pourcentage massique du composé B est de 0,1 à 5 %, la masse totale de l'électrolyte non aqueux pour une batterie au lithium-ion étant prise comme 100 %.

6. L'électrolyte non aqueux pour une batterie au lithium-ion selon la revendication 1, dans lequel le composé représenté par la formule I est choisi parmi un ou plusieurs de H₃CCOOCH₂CF₂H, H₃CH₂CCOOCH₂CF₂H, HF₂CH₂CCOOCH₃, HF₂CH₂CCOOCH₂CH₃, HF₂CH₂CH₂CCOOCH₂CH₃, H₃CCOOCH₂CH₂CF₂H, H₃CH₂CCOOCH₂CH₂CF₂H, CH₃COOCH₂CF₃, HCOOCH₂CHF₂, HCOOCH₂CF₃ et CH₃COOCH₂CF₂CF₂H.

7. L'électrolyte non aqueux pour une batterie au lithium-ion selon la revendication 1, dans lequel le composé représenté par la formule II est choisi parmi un ou plusieurs de CH₃OCOOCH₂CF₂H, CH₃OCOOCH₂CF₃, CH₃OCOOCH₂CF₂CF₂H, HCF₂CH₂OCOOCH₂CH₃ et CF₃CH₂OCOOCH₂CH₃.

8. L'électrolyte non aqueux pour une batterie au lithium-ion selon la revendication 1, dans lequel le composé A comprend en outre un composé représenté par la formule III, 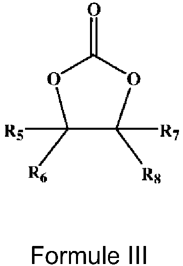 dans la formule III, R₅, R₆, R₇ et R₈ sont chacun indépendamment choisis parmi un atome d'hydrogène, un atome de fluor, un groupe hydrocarboné en C1-C4 ou un groupe fluorohydrocarboné en C1-C4, et au moins l'un de R₅, R₆, R₇ et R₈ contient un atome de fluor.

9. L'électrolyte non aqueux pour une batterie au lithium-ion selon la revendication 8, dans lequel le composé représenté par la formule III est choisi parmi un ou plusieurs des composés 10 à 13 représentés par les structures suivantes,

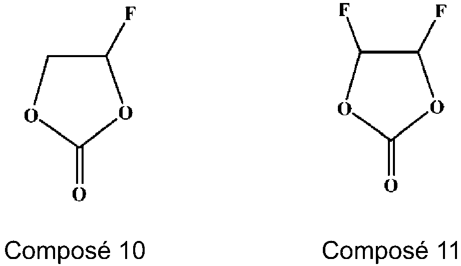

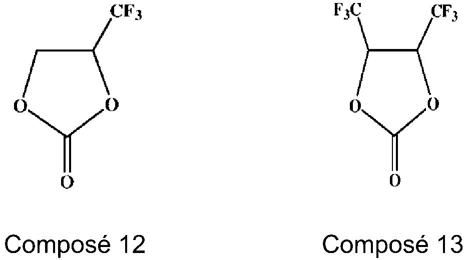

10. L'électrolyte non aqueux pour une batterie au lithium-ion selon la revendication 1, dans lequel le pourcentage massique du composé A est supérieur à 5 % et inférieur à 80 %, la masse totale de l'électrolyte non aqueux pour une batterie au lithium-ion étant prise comme 100 %.

11. L'électrolyte non aqueux pour une batterie au lithium-ion selon la revendication 8, dans lequel R₅ dans la formule III est du fluor, R₆, R₇ et R₈ sont chacun indépendamment choisis parmi un atome d'hydrogène, un atome de fluor, un groupe hydrocarboné en C1-C4 ou un groupe fluorohydrocarboné en C1-C4, et R₆, R₇ et R₈ ne peuvent pas être simultanément des atomes d'hydrogène ; ou dans lequel R₅ est un groupe hydrocarboné contenant du fluor en C1-C4, R6, R7 et R8 sont indépendamment choisis parmi un atome d'hydrogène, un atome de fluor, un groupe hydrocarboné en C1-C4 ou un groupe hydrocarboné contenant du fluor en C1-C4.

12. L'électrolyte non aqueux pour une batterie au lithium-ion selon la revendication 1, dans lequel l'électrolyte non aqueux pour ions lithium comprend en outre au moins un composé choisi parmi les composés carbonates cycliques insaturés et les composés sultones.

13. L'électrolyte non aqueux pour une batterie au lithium-ion selon la revendication 12, dans lequel le composé carbonate cyclique insaturé comprend au moins l'un du carbonate de vinylène et du carbonate de vinyl-éthylène ;
le composé sultone est choisi parmi au moins l'un de la 1,3-propane sultone, de la 1,4-butane sultone et de la 1,3-propène sultone ;
le pourcentage massique du composé carbonate cyclique insaturé est de 0,1 à 5 %, la masse totale de l'électrolyte non aqueux pour une batterie au lithium-ion étant prise comme 100 % ; le pourcentage massique du composé sultone est de 0,1 à 5 %, la masse totale de l'électrolyte non aqueux pour une batterie au lithium-ion étant prise comme 100 %.

14. Un électrolyte non aqueux pour une batterie au lithium-ion, comprenant un composé A et un composé B, dans lequel le composé A est un composé représenté par la formule structurale III suivante ; le composé B est un composé représenté par la formule structurale IV suivante, 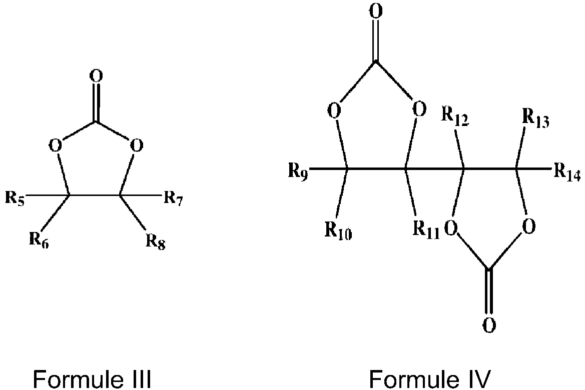
dans la formule III, R₅ est du fluor, R₆, R₇ et R₈ sont chacun indépendamment choisis parmi un atome d'hydrogène, un atome de fluor, un groupe hydrocarboné en C1-C4 ou un groupe fluorohydrocarboné en C1-C4, et R₆, R₇ et R₈ ne peuvent pas être simultanément des atomes d'hydrogène ; ou R₅ est un groupe fluoroalkyle en C1-C4, R₆, R₇ et R₈ sont indépendamment choisis parmi un atome d'hydrogène, un atome de fluor, un groupe hydrocarbyle en C1-C4 ou un groupe fluoroalkyle en C1-C4 ;
dans la formule IV, R₉, R₁₀, R₁₁, R₁₂, R₁₃ et R₁₄ sont chacun indépendamment choisis parmi un atome d'hydrogène, un atome de fluor ou un groupe en C1-C5.

15. L'électrolyte non aqueux pour une batterie au lithium-ion selon la revendication 14, dans lequel le composé représenté par la formule III est choisi parmi un ou plusieurs des composés 11 à 13 représentés par les structures suivantes, 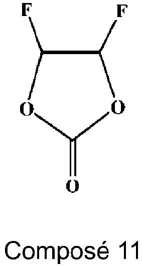 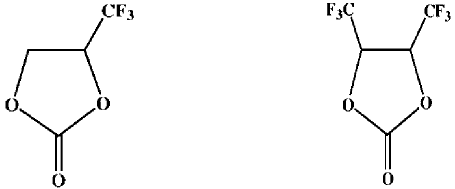 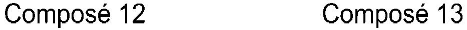 le composé B comprend un ou plusieurs des composés 1 à 9 représentés par les formules structurales suivantes,

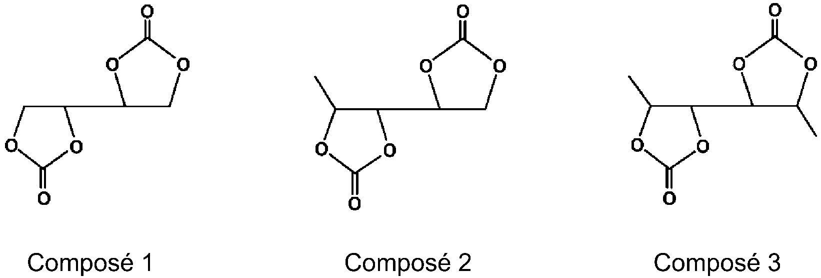

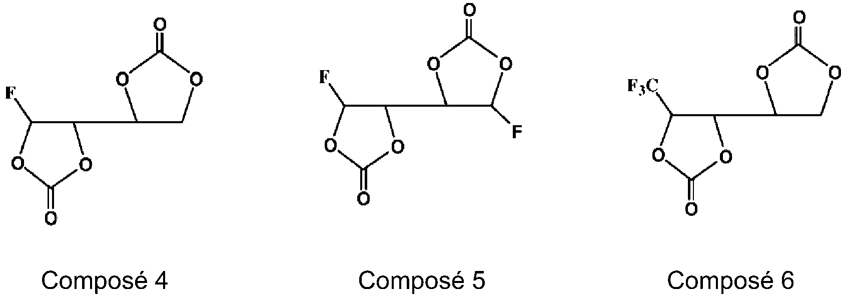

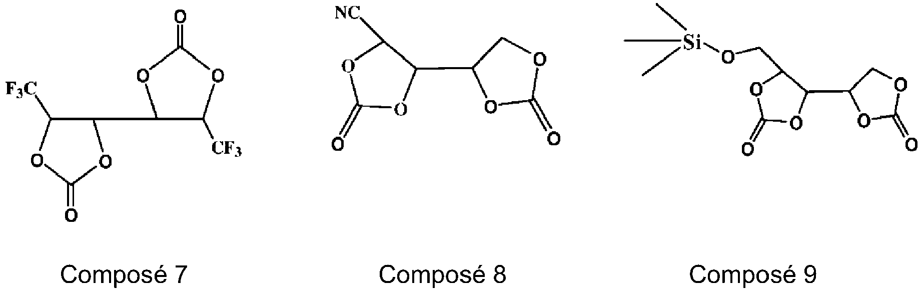

16. L'électrolyte non aqueux pour une batterie au lithium-ion selon la revendication 14, dans lequel le pourcentage massique du composé B est de 0,1 à 5 %, la masse totale de l'électrolyte non aqueux pour une batterie au lithium-ion étant prise comme 100 % ;
le pourcentage massique du composé A est supérieur à 5 % et inférieur ou égal à 80 %, la masse totale de l'électrolyte non aqueux pour une batterie au lithium-ion étant prise comme 100 %.

17. L'électrolyte non aqueux pour une batterie au lithium-ion selon la revendication 14, dans lequel l'électrolyte non aqueux pour ions lithium comprend en outre au moins un composé choisi parmi les composés carbonates cycliques insaturés et les composés sultones ;
le pourcentage massique du composé carbonate cyclique insaturé est de 0,1 à 5 %, la masse totale de l'électrolyte non aqueux pour une batterie au lithium-ion étant prise comme 100 % ; le pourcentage massique du composé sultone est de 0,1 à 5 %, la masse totale de l'électrolyte non aqueux pour une batterie au lithium-ion étant prise comme 100 %.

18. Une batterie au lithium-ion, comprenant une électrode positive, une électrode négative, un séparateur interposé entre l'électrode positive et l'électrode négative, et un électrolyte, dans laquelle l'électrolyte est l'électrolyte non aqueux pour une batterie au lithium-ion selon l'une quelconque des revendications 1 à 17.

19. La batterie au lithium-ion selon la revendication 18, dans laquelle l'électrode positive comprend un matériau actif d'électrode positive, et le matériau actif d'électrode positive est au moins l'un de LiNiₓCo_{y}Mn_{z}L_{(1-x-y-z)}O₂, LiCo_{x'}L_{(1-x')}O₂, LiNi_{x"}L'_{y}'Mn_{(2-x"-y')}O₄ et Li_{z'}MPO₄, dans laquelle L est au moins l'un de Al, Sr, Mg, Ti, Ca, Zr, Zn, Si ou Fe, 0≤x≤1, 0≤y≤1, 0≤z≤1, 0<x+y+z≤1, 0<x'≤1, 0.3≤x"≤0.6, 0.01≤y'≤0.2, L' est au moins l'un de Co, Al, Sr, Mg, Ti, Ca, Zr, Zn, Si et Fe ; 0.5≤z'≤1, M étant au moins l'un de Fe, Mn et Co.
